(19) Europäisches Patentamt
European Patent Office
Office européen des brevets

(11) EP 3 357 903 B1

(12) EUROPEAN PATENT SPECIFICATION

(45) Date of publication and mention of the grant of the patent:
**04.11.2020 Bulletin 2020/45**

(21) Application number: **17154882.9**

(22) Date of filing: **06.02.2017**

(51) Int Cl.:
*C07C 67/58* (2006.01) *C07C 67/293* (2006.01)
*C07C 69/145* (2006.01) *C07C 69/16* (2006.01)
*C07C 67/333* (2006.01) *C07C 69/753* (2006.01)
*C07C 69/757* (2006.01) *C07D 317/44* (2006.01)

(54) **METHOD FOR A CONTINUOUS PRODUCTION OF A Z-CYCLOOCTENE**

VERFAHREN ZUR KONTINUIERLICHEN HERSTELLUNG EINES Z-CYCLOOCTENS

PROCÉDÉ POUR UNE PRODUCTION EN CONTINU D’UN Z-CYCLOOCTÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.08.2018 Bulletin 2018/32**

(73) Proprietor: **Stichting Katholieke Universiteit
6525 EZ Nijmegen (NL)**

(72) Inventors:
- **RUTJES, Floris Petrus Johannes Theodorus
6500 HC Nijmegen (NL)**
- **BLANCO ANIA, Daniel
6500 HC Nijmegen (NL)**
- **MAARTENSE, Luuk
6500 HC Nijmegen (NL)**

(74) Representative: **EDP Patent Attorneys B.V.
Bronland 12-E
6708 WH Wageningen (NL)**

(56) References cited:
**EP-A1- 0 402 744 US-A- 3 848 011**

- **ROYZEN M ET AL: "A photochemical synthesis of functionalized trans-cyclooctenes driven by metal complexation", JOURNAL OF THE AMERICAN CHEMICAL SOCIETY, AMERICAN CHEMICAL SOCIETY, US, vol. 130, no. 12, 26 March 2008 (2008-03-26), pages 3760-3761, XP002629676, ISSN: 0002-7863, DOI: 10.1021/JA8001919 [retrieved on 2008-03-06]**
- **ARTHUR C COPE ET AL: "trans-CYCLOOCTENE", ORGANIC SYNTHESES., vol. 49, 1 January 1969 (1969-01-01), page 39, XP055387560, US ISSN: 0078-6209, DOI: 10.15227/orgsyn.049.0039**
- **Eckart Müller ET AL: "Liquid-Liquid Extraction" In: "Ullmann’s Encyclopedia of Industrial Chemistry", 15 January 2008 (2008-01-15), Wiley-VCH, Weinheim, XP055387945, ISBN: 978-3-527-30673-2 DOI: 10.1002/14356007.b03_06.pub2, * the whole document ***

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 357 903 B1

## Description

FIELD OF THE INVENTION

The invention relates to a continuous method for generating a first compound, wherein the first compound is an E-cyclooctene. The invention further relates to the use of a a system for continuously producing such a first compound.

BACKGROUND OF THE INVENTION

Methods and devices to produce *E*-isomers of alkenes are known in the art. In e.g. M. Royzen et al., vol. 130 of the Journal of the American Chemical Society of 2008 (pp. 3760-3761) the synthesis of *trans*-cyclooctenes by photo-isomerization of *cis*-cyclooctenes is described, wherein a mixture of *trans*-cyclooctenes is formed, with a successive trapping of the *trans*-cyclooctenes on a silver nitrate impregnated silica column. The *cis*-isomer successively elutes back to the reaction flask and is further circulated through the system and may be isomerized again. After complete consumption of the *cis*-cyclooctene, the silica is removed and stirred with $NH_4OH$, which liberates the *trans*-cyclooctene from the $AgNO_3$. The *trans*-cyclooctene derivative is then recovered by extraction.

Arthur C. Cope et al., "trans-cyclooctene", Organic synthesis (1969), vol. 49, pp. 39-40 describe a batch process for the synthesis of a mixture of *cis*- and *trans*-cyclooctene, and the use of an aqueous silver nitrate solution in the successive batch extraction carried out in a separatory funnel. Separation of *trans*-cyclooctene from the mixture of *trans*- and *cis*-cyclooctene in an organic solution is performed by extraction with an aqueous silver nitrate solution. Separated *trans*-cyclooctene is isolated from the aqueous solution using ammonium hydroxide.

SUMMARY OF THE INVENTION

Bioorthogonal ligation reactions are broadly used in a wide variety of applications in the fields of chemistry, materials science, drug discovery, drug delivery, (pre)clinical imaging and molecular biology. In case ligation reactions proceed spontaneously and have high reaction rates (so-called click reactions), those bioorthogonal reactions can be used (both in vitro and in vivo) to functionalize (bio)molecules at low concentrations under mild conditions. The [4+2] inverse-electron-demand Diels-Alder cycloaddition of tetrazines with *E*-cyclooctene (also referred to as "*trans*-cyclooctene") derivatives, e.g., has a reaction rate up to 2000 $M^{-1}s^{-1}$ and therefore fulfills one of the most important requirements for in vitro or in vivo bioorthogonal ligation.

A direct route to synthesize *E*-cyclooctenes is by photo-isomerization of *Z*-cyclooctenes ("*cis*-cyclooctenes") with ultraviolet light (UV light) with a wavelength in the range of 254 nm. Exposure of Z-cyclooctene to such UV light leads to partial isomerization and results in an equilibrium between the *E*/*Z*-isomers. Typically, only a small fraction of the *Z*-cyclooctene is isomerized to the *E*-isomer, with the isomerization rate and efficiency depending on the residence time, the sensitizer (see below) used and the substituents attached to the Z-cyclooctene (derivative).

Methods and devices to produce *E*-isomers of alkenes, such as *E*-cyclooctene, may require additional unit operations to obtain the target *E*-cyclooctenes. Furthermore, such methods may not be continuous and may not be easily scaled-up. Further, such solutions may not be efficient. Especially, the silica gel used in prior art solutions appears to become saturated in time.

Hence, it is an aspect of the invention to provide an alternative method and/or use of a system, that preferably further at least partly obviate(s) one or more of the above-described drawbacks. The present invention may have as object to overcome or ameliorate at least one of the disadvantages of the prior art, or to provide a useful alternative. The invention is defined by the claims. Any subject-matter falling outside the scope of the claims is provided for information purposes only.

In a first aspect, the disclosure provides a method for a continuous production of (a product comprising) a first compound, the method comprising continuously providing a first liquid to a mixing zone in an extraction unit, wherein the first liquid comprises the first compound and a second compound, contacting, especially mixing, in the extraction unit, especially in the mixing zone, the first liquid with a second liquid, wherein the first liquid and the second liquid are not miscible, wherein the first compound, optionally in combination with a (optional) third compound, has a higher solubility in the second liquid than the second compound optionally in combination with the (optional) third compound, wherein in a first withdrawal zone in the extraction unit, in fluid contact with the mixing zone, at least part of the first liquid or the second liquid, especially, the first liquid, is (continuously) removed from the extraction unit.

Especially, the first compound and the second compound may be an (*E* and respectively *Z*) isomer (of an alkene (olefin)). The first and second compound may (respectively) be a substituted *E*-isomer and a substituted *Z*-isomer (or *cis* and *trans*) isomer of the same alkene (or respectively a substituted *Z* and *E* isomer of the same alkene). Especially, the first compound is a (substituted) *E*-isomer of an alkene (olefin), and the second compound is a (corresponding) (substituted) *Z*-isomer of said alkene. The first liquid especially is an organic liquid (herein also indicated as "first organic

liquid") and the second liquid is especially an aqueous liquid. The (optional) third compound may for instance be metal salt. The first compound may be obtained in the second liquid (after production of the first compound).

The *E*-isomer (of an alkene) may comprise an *E*-cycloalkene and/or a substituted *E*-cycloalkene. Accordingly, especially, the (corresponding) *Z*-isomer comprises a (corresponding) *Z*-cycloalkene or substituted *Z*-cycloalkene (of said alkene). Especially, the *E*-isomer comprises *E*-cyclooctene or a substituted *E*-cyclooctene.

Herein the term "substituted" such as in "a substituted cycloalkene" relates to a compound (such as cycloalkene) wherein one or more hydrogen atoms is/are substituted by one or more other groups, e.g. by an alkyl group, and acyloxy group or a (further) cyclic group (see further below). Especially, herein the term "isomer" relates to an "isomer of an alkene ("olefin")". Especially the isomer comprises a dienophile isomer, i.e. a compound that may readily react with a diene, especially in a Diels-Alder reaction.

Herein, the term "continuous production of a first compound" may relate to the production of the first compound as such. The continuous production of a first compound may (also) relate to the continuous production of a product comprising the first compound. The product may e.g. comprise a complex comprising the first compound. The product may further comprise a liquid wherein the first compound (or a complex comprising the first compound) is dissolved in said liquid.

In the method of the invention (and also the system used according to the invention, see further below) isomers and their substituted isomers may behave substantially the same, especially with respect to an extraction in the extraction unit, and especially with respect to an isomerization - in an isomerization stage (see below). Unless stated otherwise, herein, the term "isomer" relates to the (unsubstituted) "isomer" and/or the "substituted isomer". Hence, when the term "and/or substituted in combination with a *specific* isomer" such as in "an *E*-isomer and/or substituted *E*-isomer" and "a *Z*-cyclooctene and/or substituted *Z*-cyclooctene" is not mentioned in the description, this does not imply that the substituted isomer(s) is (are) not covered by the invention. Especially, the terms like "isomer", "*E*-isomer", "cyclooctene" and the like may relate to the (non-substituted) "isomer" (or "*E*-isomer, "cyclooctene") and the like and/or to a corresponding "substituted isomer" ("substituted *E*-isomer", "substituted cyclooctene") and the like. For instance, an *E*-cyclooctene may comprise an (*E*)-cyclooct-2-en-1-yl alkanoate (or, e.g., an (*E*)-cyclooct-2-en-1-yl arenecarboxylate), an (*E*)-cyclooct-4-en-1-yl alkanoate (or a corresponding arenecarboxylate), (*E*)-cyclooct-2-en-1,4-diyl dialkanoate (or a corresponding diarenecarboxylate), as well as non-substituted *E*-cyclooctene. An *E*-cyclooctene may in embodiments comprise (*E*)-10,10-dimethyl-9,11-dioxabicyclo[6.3.0]undec-4-ene, and/or an organyl (*E*)-bicyclo[6.1.0]non-4-ene-9-carboxylate, e.g., ethyl (*E*)-bicyclo[6.1.0]non-4-ene-9-carboxylate. (see also other compounds described in the application). Especially the corresponding *Z*-isomer comprises a (corresponding) *Z*-cyclooctene and/or substituted *Z*-cyclooctene.

Hence, herein the terms "cycloalkene" and "cyclooctene" used in "*E*-cycloalkene", "*Z*-cycloalkene", "cyclooctenes" "*E*-cyclooctene", "*Z*-cyclooctene", "*cis*/*trans*-cyclooctenes" and the like, especially relate to the compounds "cycloalkene" and "cyclooctene" as well as a substituted cycloalkene, especially a substituted cyclooctene. Likewise, the terms "(substituted) isomer" and the like may relate to said "(unsubstituted) isomer" and/or said "substituted isomer". Especially, the isomers of cyclooctene may be substituted analogously to isomers of cyclooctyne, such as for instance being described in EP 2894 142 A1.

Cyclooctenes (*E*-isomers) produced according to the invention may relate to the compounds having a general formula (XI), (XII), (XIII), (XIV), (XV), and (XVI) depicted in Fig. 3 comprising a cyclooctene ring comprising two carbons joined to a double bound, and wherein:

X and X' are independently selected from the group consisting of hydrogen, halogen, -OR, $-C(O)_2R^2$, $-NO_2$, -CN, $-S(O)_2R$, $C_1$ - $C_{12}$ alkyl groups, $C_1$ - $C_{12}$ aryl groups, $C_1$ - $C_{12}$ alkylaryl groups, and $C_1$ - $C_{12}$ arylalkyl groups, wherein the alkyl groups, aryl groups, alkylaryl groups and arylalkyl groups are optionally substituted, and wherein R is independently selected from the group consisting of hydrogen, halogen, $C_1$ - $C_{24}$ alkyl groups, $C_6$ - $C_{24}$ (hetero)aryl groups, $C_7$ - $C_{24}$ alkyl(hetero)aryl groups and $C_7$ - $C_{24}$ (hetero)arylalkyl group, wherein the alkyl groups, (hetero)aryl groups, alkyl(hetero)aryl groups and (hetero)arylalkyl groups are independently optionally substituted with one or more substituents independently selected from the group consisting of $C_1$ - $C_{12}$ alkyl groups, $C_2$ - $C_{12}$ alkenyl groups, $C_2$ - $C_{12}$ alkynyl groups, $C_3$ - $C_{12}$ cycloalkyl groups, $C_1$ - $C_{12}$ alkoxy groups, $C_2$ - $C_{12}$ alkenyloxy groups, $C_2$ - $C_{12}$ alkynyloxy groups, $C_3$ - $C_{12}$ cycloalkyloxy groups, halogens, amino groups, oxo groups and silyl groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S, wherein the silyl groups are represented by the formula $(R^3)_3Si$-, wherein $R^3$ is independently selected from the group consisting of $C_1$ - $C_{12}$ alkyl groups, $C_2$ - $C_{12}$ alkenyl groups, $C_2$ - $C_{12}$ alkynyl groups, $C_3$ - $C_{12}$ cycloalkyl groups, $C_1$ - $C_{12}$ alkoxy groups, $C_2$ - $C_{12}$ alkenyloxy groups, $C_2$ - $C_{12}$ alkynyloxy groups and $C_3$ - $C_{12}$ cycloalkyloxy groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S, and $R^2$ is independently selected from the group consisting of $C_1$ - $C_{24}$ alkyl groups, $C_6$ - $C_{24}$ (hetero)aryl groups, $C_7$ - $C_{24}$ alkyl(hetero)aryl groups and $C_7$ - $C_{24}$ (hetero)arylalkyl groups, and wherein the alkyl groups, (hetero)aryl groups, alkyl(hetero)aryl groups and (hetero)arylalkyl groups are optionally substituted;

R is as defined above; and wherein

X can be located at any position on the cyclooctene ring other than at the two carbons joined to the double bound, and wherein an X (at any of an unsubstituted sp3 carbon atoms in the ring) is optionally linked to a carbon atom in the -OR group, forming a cyclic group. Such link may only be formed with an atom in X that may form a bound with said carbon atom.

The ring of the compounds depicted with formula (*XI*), (*XII*), and (*XIII*) may comprise 13 moieties (or groups) next to the OR group. Hence n may be at maximum 13. In embodiments, X is linked to a carbon atom in the OR group, see e.g. the compounds depicted by formula (*I*) and formula (*X*) in Fig. 3. Hence, embodiments of the compound depicted with formula (*XI*), (*XII*), and (*XIII*) may comprise 12 moieties next to the OR group. The ring of the compounds depicted with formula (*XIV*), (*XV*), and (*XVI*) may comprise 12 moieties (or groups) next to the cyclopropyl group. Hence, n may also be at maximum 12. Herein a moiety may relate to a group having 1 atom, (such as hydrogen or a halogen) as well as a group comprising more than one atom. Especially a number of additional moieties may be defined by the number of moieties next to the OR group respectively next to the cyclopropyl group. Especially, n may relate to the number of additional moieties. Especially, all additional moieties comprise X, wherein X is independently selected from the group consisting of hydrogen, OH, and halogen. Especially all additional moieties comprise hydrogen. In embodimements (of the first compound) at least one additional moiety comprises more than one atom, especially at least 3 atoms, such as at least 4 atoms. Such additional moiety may especially be bound to a C-atom in the ring, wherein said C-atom does not comprise the double bound or the OR group or the cyclopropyl group.

In further embodiments (of the first compound), at least two (additional) moeities comprise at least 3 atoms, such as at least 4 atoms. Especially, said two moieties are not bound to the same C-atom in the ring. Especially, the isomer (the first compound) comprises less than or equal to 6, especially less than or equal to 5, even more especially equal to or less than 4, moieties comprising at least 4 atoms. Especially, the number of moieties (of the isomer / first compound) comprising at least 3 atoms, especially at least 4 atoms, is selected in the range of 0-4. Especially, the number of moieties (comprised by the first compound) not being hydrogen, halogen, or OH is at maximum 4.

The "undulated" (or "wiggled") bond in the figure schematically depicts a substituent that may be arranged at either sides of the ring structure, especially indicating (two) different diastereoisomers (or "diastereomers"). Herein the terms "Me", "Et", "Bu", "Pr", "Cy", "Ph", "Tol", "Pyr" will be understood by the skilled person and relate to "methyl", "ethyl", "buthyl", "propyl" "a cyclic group", "phenol", "toluene", "pyridine", respectively.

Also disclosed herein are isomers comprising (substituted) cyclic alkenes, which may comprise more than 8 carbon atoms in the ring (alternatively or additionally to cyclooctene). Said isomers may be substituted analogeously to the cyclocotenes as described above.

Furthermore, the term "(substituted) *E*-cyclooctene" may also be indicated by the term "ECO". Likewise, the term "(substituted) *Z*-cyclooctene" may also be indicated by the term "ZCO" The term "(substituted) *cis*-cyclooctene" may also be indicated by the term "CCO", and "(substituted) *trans*-cyclooctene" may also be indicated by the term "TCO". Further, the terms "alkene" and "olefin" may be used interchangeably.

Especially, the extraction (unit) comprises a liquid-liquid extraction (unit), wherein (during operation) the first liquid contacts the second liquid and especially wherein the (first and the second) liquids are immiscible. The first liquid is an organic liquid, e.g. selected from the group consisting of hydrocarbons (e.g., pentane, hexane, heptane, benzene, toluene, xylene). The first liquid may further relate to a combination of more than one (different) first liquids. Especially, the organic liquid comprises heptane, especially n-heptane.

The second liquid is an aqueous liquid. Such aqueous liquid may be substantially water. Such aqueous liquid may also comprise a mixture comprising water and an alcohol or other (polar) elements (being soluble in water).

Hence, the first liquid may in embodiments comprise one or more apolar liquids, such as one or more of pentane, cyclopentane, hexane, cyclohexane, benzene, toluene, 1,4-dioxane, chloroform, diethyl ether, dichloromethane (DCM), etc.. The second liquid may comprise a (polar) liquid, such as one or more of tetrahydrofuran (THF) ethyl acetate, acetone, dimethylformamide (DMF), acetonitrile (MeCN), dimethyl sulfoxide (DMSO), nitromethane, and propylene carbonate. In yet other embodiments, the second liquid may comprise a (polar) liquid, such as one or more of acetic acid, n-butanol, isopropanol, n-propanol, ethanol, methanol, formic acid, and water, especially water. Combinations of two or more (first or second) liquids may also be applied.

The first compound may translocate in a larger extend from the first liquid to the second liquid, relative to the second compound. Advantageously, with this method, the first compound may be trapped in the second liquid and especially may be easily isolated from the second liquid. Such translocation from the first liquid to the second liquid may further be improved by (providing) a third compound in (to) the second liquid which interacts with the first compound,

especially to keep the first compound in the second liquid (see further below).

Advantageously, with the method the first compound may be produced in a continuous way. Especially, the method does not require additional steps to liberate the first compound from any gel, such as a silica gel, or removing/refreshing a silica gel. The method may allow easily removing of the second liquid (comprising the first compound) from the extraction unit and/or replacing the second liquid (comprising the first compound) with "fresh" second liquid, e.g. when the second liquid may be saturated with the first compound (see further below). Especially, the method allows scaling up. The term "producing" may thus also refer to providing in a higher purity and/or isolating.

Herein the term "solubility" especially relates to a maximum amount (of moles) of a compound that can dissolve in a specific volume of a liquid. Hence, the phrase "wherein the first compound, optionally in combination with a third compound, has a higher solubility in the liquid than the second compound optionally in combination with the third compound" and similar phrases especially relates to a condition wherein a ratio of a maximum amount (of moles) of the first compound that may dissolve (either directly or in combination with a third compound) in a specific volume of the liquid (relative) to a maximum amount (of moles) of the second compound that may dissolve (either directly or in combination with the third compound) in the (same) specific volume of the liquid is larger than 1, especially equal to or larger than 2, such as equal to or larger than 5. Especially, said ratio is equal to or larger than 10. Even more especially, said ratio is equal to or larger than 100.

In embodiments, the *E*-isomer (in the first liquid) may contact the third compound, (in the second liquid) at a liquid interface (especially at a liquid-liquid interface) between the first liquid and the second liquid (when in the extraction unit the first liquid and the second liquid are contacted, especially mixed) and may form a complex with the third compound. Especially, the second compound may essentially not form a complex with the optional third compound. In such embodiment the first compound (especially, the complex of the first compound and the third compound) may dissolve to a larger extend in the second liquid relative to the second compound. In such embodiments, said ratio may especially be equal to or larger than 10, especially equal to or larger than 100.

Especially, the third compound may form a complex with first compound. The third compound may be a metal salt. Especially the third compound comprises a metal cation. The metal cation may e.g. comprise $Ni^{2+}$, $Cu^{2+}$, $Pd^{2+}$, $Fe^{2+}$, $Mn^{2+}$, and/or $Ag^{+}$. The third compound may further comprise a counter ion (anion), e.g. a chloride, a bromide, a nitrate, or a perchlorate. The third compound may be a copper salt, such as cupper chloride, or copper nitrate. Additionally or alternatively, the third compound may comprise a silver salt, such as silver chloride, silver perchlorate, or silver nitrate. According to the invention, the third compound is silver nitrate.

Hence, the term "metal salt" may refer to the salt, but also to the salt in the solved state (such as in water), i.e. metal ions and anions of the metal salt. When forming a complex, the metal ion or anion may form a complex, or both may form a complex. Especially, at least the metal ion may be used to form a complex. Hence, the phrase "in combination with a third compound" may also refer to the presence of the metal ion of the metal salt and/or the presence of an anion, when the third compound is a metal salt, which may at least partly be solved (in e.g. water).

Essentially, in the extraction unit a transport of the first compound from the first liquid to the second liquid may be present. This may be concentration driven, as the first liquid initially comprises the first compound and the second initially (substantially) does not. A rate of such (mass) transport and/or an amount of transported first compound may among others be increased by one or more of increasing the contact area between the first liquid and the second liquid (especially by changing the efficiency of mixing in the mixing zone), increasing a concentration of the first compound in the first liquid (especially in the mixing zone), and decreasing a concentration of the first compound in the second liquid (especially in the mixing zone). Such transport (rate) may further be affected by a temperature, or a pressure of the first and/or second liquid, and/or a residence time in the extraction unit.

In embodiments at least part of the first liquid is removed from the extraction unit, especially from the mixing zone. Additionally or alternatively, at least part of the second liquid is removed from the extraction unit, especially from the mixing zone. Especially, at least one of the first liquid and the second liquid is (continuously) removed from the mixing zone, especially by removing said liquid in the first withdrawal zone being in fluid contact with the mixing zone. In further embodiments, in a second withdrawal zone in the extraction unit, in fluid contact with the mixing zone, at least part of the second liquid is removed from the extraction unit. At least part of the first liquid may (continuously) be removed in the first withdrawal zone.

In further embodiments, especially wherein the first liquid is removed from the extraction unit, the method further comprises reintroducing the first liquid (in) to the mixing zone. Hence, the first liquid may be circulated in the method, especially through the extraction unit. The first liquid may comprise a circulating first liquid.

Advantageously, a circulated first liquid may be enriched with the first compound before reintroducing the first liquid to the mixing zone. Hence, at least part of the first compound in the (circulating) first liquid may be removed (extracted) from the first liquid in the extraction unit, and especially the (circulating) first liquid may be enriched (again) with the first compound (upstream or downstream of the extraction unit). Enriching may relate to (directly) supplying an amount of the first compound to the first liquid. Alternatively or additionally, the (circulating) first liquid may be enriched with the first compound in an isomerization stage, especially wherein the second compound is (at least partly) isomerized

(converted) to the first compound, especially in an isomerization reactor (see below). Hence, the method may (further) comprise an isomerization stage (see further below).

Yet, in further embodiments, the second liquid may (also) be "refreshed". Especially a flow comprising the second liquid may be provided to the extraction unit, either continuously or e.g. in regular or irregular intervals. Especially, a concentration of the first compound in the extraction unit may be decreased (diluted) by the second liquid provided to the extraction unit. Advantageously, providing the second liquid to the extraction unit may increase the transport (translocation) of the first compound from the first liquid to the second liquid (in the extraction unit).

Hence, in embodiments, the method further comprises: providing the second liquid to the extraction unit in an introduction zone of the extraction unit, wherein the introduction zone is in fluid contact with the mixing zone. Especially, such embodiment may be advantageously combined with embodiments comprising removing the second liquid (from the extraction unit). In embodiments, the second liquid may be removed from the extraction unit in the first withdrawal zone. Hence, in further embodiments, (also) at least part of the second liquid is removed from the extraction unit. Advantageously, embodiments comprising removing of the second liquid from the extraction unit may be combined with embodiments comprising removing of the first liquid from the extraction unit. Especially, the second liquid may be removed in the second withdrawal zone (wherein the first liquid is extracted in the first withdrawal zone), especially wherein the second liquid is provided (to the extraction unit) in the introduction zone. The first liquid may especially be circulated, and part of the first liquid may especially be (continuously) removed from the first withdrawal zone.

Especially, if both liquids (i.e. the first liquid and the second liquid) are removed from the extraction unit, one of the (first and second) liquids is removed in the first withdrawal zone and the other liquid may be removed in a second withdrawal zone, (also) being in fluid contact with the mixing zone. Hence in embodiments, the first liquid may be removed (from the extraction unit) in the first withdrawal zone, wherein the second liquid is removed (from the extraction unit) in the second withdrawal zone. Yet, in other embodiments, the second liquid is removed (from the extraction unit) in the first withdrawal zone, wherein the first liquid is removed (from the extraction unit) in the second withdrawal zone. Especially, removing of the respective liquid (the first or the second liquid) from the first withdrawal zone comprises a continuous removing of said liquid.

Hence, in embodiments, in a second withdrawal zone in the extraction unit, in fluid contact with the mixing zone, at least part of the second liquid or the first liquid is continuously removed from the extraction unit, wherein the second liquid is removed from the extraction unit.

The terms "first withdrawal zone" and "second withdrawal zone" especially indicate the liquid is removed at different positions from the extraction unit. Hence, an inlet for withdrawal in a first withdrawal zone is at a different location than an inlet for withdrawal in a second withdrawal zone.

Herein, the terms "upstream" and "downstream" of a first location especially relate to a further location (i.e. an upstream location or a downstream location) relative to the first location, wherein during normal operation a flow flows from the upstream location to (and/or in the direction of) the first location. Likewise, such flow may flow from the first location to (and/or in the direction of) the downstream location. Further, especially in a circulating flow a location may be downstream of a location and also upstream of the same location. For instance, a liquid at a location between the extraction unit and the isomerization reactor may flow from the extraction unit (thus being "downstream" of the extraction unit at said location) via the isomerization reactor and again to the extraction unit (and thus also being "upstream" of the extraction unit.

The term "downstream of the extraction unit" especially relates to a location at which the liquid exits the extraction unit up to, and including any location in the isomerization reactor (and thus including any location in between). The term upstream of the extraction unit especially relates to any location in the isomerization stage up to a location at which the liquid enters the extraction unit. Likewise, a location upstream of the isomerization reactor especially relates to any location in the extraction unit up to a location at which the liquid may enter the isomerization reactor (during normal operation). And especially a location downstream of the isomerization reactor may relate to any location at which the liquid may exit the isomerization reactor up to and including any location in the extraction unit (during normal operation). Hence, a location upstream of the extraction unit and downstream of the isomerization unit (and the like), especially relates to a location at which the liquid may exit the extraction unit up to a location at which the liquid may enter the isomerization unit (and the like) (during normal operation).

In the isomerization stage, a *Z*-isomer, such as *Z*-cyclooctene (or an *E*-isomer) may be converted (isomerized) into a mixture of said *Z*-isomer and corresponding *E*- isomer. In the isomerization stage an equilibrium may be established between the *E* and *Z* isomers when the *Z*-isomer (or *E*-isomer) is isomerized. Especially, a concentration of an *E*-isomer (or a *Z*-isomer) (especially, the first compound) in the first liquid may increase in the isomerization stage. Hence when the first liquid is reintroduced to the mixing zone, the concentration of the *E*-isomer (or *Z*-isomer) (the first compound) in the first liquid may be larger than said concentration after removing the first liquid from the extraction unit (and before the isomerization stage), i.e. downstream from the extraction unit. A second concentration of the first compound at a location downstream of the isomerization reactor, wherein the isomerization stage takes place, (and upstream of the extraction unit) (or in the isomerization reactor) may by larger than a first concentration upstream of the isomerization

reactor (and downstream of the extraction stage). In embodiments, e.g., a concentration of *E*-cyclooctene is increased in the isomerization stage, especially wherein a concentration of *Z*-cyclooctene decreases in the isomerization stage (due to the isomerization to the *E*-cyclooctene). Especially, in such embodiment a further amount of the *E*-isomer (or *Z*-isomer) (first compound) may be transported from the first liquid to the second liquid when the first liquid and the second liquid are contacted (again) (in the extraction unit).

Hence, in embodiments, especially wherein the first liquid is removed from the extraction unit, the method further comprises reintroducing the first liquid to the mixing zone after subjection of the first liquid to an isomerization stage wherein the first liquid is enriched with the first compound (by a, at least partly, isomerization of the second compound). The isomerization may especially be provided, in an isomerization reactor, external from the extraction unit (see also below).

Yet, in further embodiments, the extraction unit may comprise an isomerization zone (for providing the isomerization). Hence, in embodiments, the extraction unit comprises the isomerization zone (wherein a feed of the isomerization zone is in fluid contact with the first removal zone and a discard of the isomerization zone is in fluid contact with the mixing zone). Especially the isomerization zone comprises the flow channel (see further below). Hence, the extraction unit may further comprise an isomerization reactor.

Further, additionally an amount of the second compound may be provided to the first liquid, especially to further enrich the first liquid with the first compound (when at least part of the second compound is converted in the isomerization stage into the first compound). Especially, the second compound may be provided in the first liquid at a location upstream of the isomerization reactor (, wherein the isomerization stage takes place). In embodiments, especially comprising a circulating first liquid, the first liquid may be enriched with the second compound at a location downstream of an isomerization reactor, wherein the isomerization stage takes place, and especially upstream of the extraction unit. In yet further embodiments, the first liquid may be enriched with the second compound at a location upstream of the extraction unit.

Hence, in further embodiments, the first liquid may (also) be enriched with the second compound. Especially, enriching the first liquid with the second compound comprises providing an amount of the second compound to the first liquid, especially wherein a concentration of the second compound in the first liquid increases (upstream of the isomerization stage). Successively, in the isomerization stage an increased concentration of the second compound in the first liquid may provide an increased concentration of the first compound in the first liquid, especially wherein the isomerization comprises an equilibrium reaction between the isomers (in the isomerization stage, and especially downstream of the isomerization reactor and, especially, upstream of the extraction unit).

The isomerization (in the isomerization stage) comprises photo-isomerization. Photo-isomerization may advantageously be used for isomerization because of its controllability. The isomerization reaction may start when the light is provided and stop when no light is provided (anymore). Photo-isomerization may comprise providing light to the first liquid, especially visual light and/or ultraviolet (UV) light. Hence, photo isomerization may comprise light having a wavelength selected in the range of 100-1000 nm, especially in the range of 100-700 nm.

The method, especially photo-isomerization may comprise providing ultraviolet (UV) light to the first liquid. In embodiments, the UV-light comprises a wavelength selected in the range of 100-280 nm, such as selected in the range of 240-270 nm. Especially, the wavelength may comprise 254 nm ($\pm$ 5 nm). In embodiments, the isomerization stage comprises subjecting the first liquid to (UV) light, especially external from the extraction unit. In further embodiments, subjecting the first liquid to (UV) light may be provided in the extraction unit, especially in the isomerization unit (of the extraction unit).

Photo-isomerization may further be facilitated by a (photo)sensitizer provided to the first liquid. Especially, the sensitizer may advantageously positively affect the rate of conversion of the first (*E* or *Z*) isomer into the corresponding (*Z* or *E*) isomer. The sensitizer may further affect the equilibrium between the *E* and *Z* isomer. Hence, in embodiments, the first liquid further comprises a sensitizer. The sensitizer may be selected based on the isomer to be converted. In embodiments, the sensitizer is selected from the group consisting of acetophenone, benzophenone, benzil, acridine, duroquinone, pyrene, eosin, and an organyl benzoate, especially methyl benzoate. In embodiments, the sensitizer comprises an organyl benzoate, especially methyl benzoate. Especially, the first liquid comprises a (photo)sensitizer comprising a sensitizer concentration relative to a volume of the first liquid selected in the range of 1-100 mM, such as 10-75 mM, especially selected in the range of 10-40 mM, such as 15-25 mM. Especially, the sensitizer comprises methyl benzoate.

In further embodiments, the photo-isomerization may be provided by visible light (and near IR), especially comprising a wavelength selected in the range of 380-800 nm, even more especially in the range of 420-500 nm. This (photo-isomerization) process may be facilitated by a metal complex provided to the first liquid. Especially, coordination of such metal complex to the olefin may advantageously affect the rate of conversion of the first (*E* or *Z*) isomer into the corresponding (*Z* or *E*) isomer. The metal complex may further affect the equilibrium between the *E* and *Z* isomer. The metal complex may function as a (photo) sensitizer. Especially, the (photo) sensitizer is a metal complex. Hence, in embodiments, the first liquid further comprises a metal complex, especially contains a metal complex. The metal complex may be selected based on the isomer to be converted. Especially, the metal complex comprises a transition-metal

complex. In embodiments, the metal complex is selected from the group consisting of Ru(II)-based metalorganic complexes. The metal complex may comprise a $Ru^{II}$ bipyridine (bpy) complex, especially $[Ru(bpy)_2](PF_6)_2$. Especially, the first liquid comprises a metal complex comprising a metal complex concentration relative to the amount of the second compound in the first liquid (in the isomerization stage) selected in the range of 0.01-1.0 mol%.

Subjecting the first liquid to (UV) light or other energy sources may heat the first liquid. Hence in embodiments, the method further comprises cooling the first liquid. Cooling of the first liquid may be provided at different locations in the system comprising the extraction unit and the isomerization reactor. Cooling may be provided (to the first liquid) at the isomerization reactor. Cooling (of the first liquid) may be provided at the extracting unit. The system may comprise a cooling system (providing the cooling to of the first liquid). Especially, the cooling system is configured at a location upstream of the extraction unit. In embodiments the cooling system is arranged at the isomerization reactor and configured to cool the first liquid in the isomerization reactor. Hence, the isomerization stage may further comprise cooling the first liquid. In further embodiments the cooling system is arranged at a location downstream to the isomerization reactor and upstream to the extraction unit (and configured to cool the first liquid downstream of the isomerization reactor and upstream of the extraction unit.

Advantageously, the method of the invention (and also the system used according to the invention) may be used to provide (substituted) *E*-cyclooctene(s). In embodiments, the first compound and the second compound comprise respectively an (*E*)-cyclooctene ester or a protected (*E*)-cyclooctenediol and its corresponding (*Z*)-cyclooctene ester or protected (*Z*)-cyclooctenediol. In embodiments, the first compound comprises one or more compounds selected from the group consisting of a cyclooctene ester or a protected cyclooctenediol. Especially, the second compound comprises a respective isomer of the first compound. In embodiments, the cyclooctene ester comprises a cyclooctenyl alkanoate. In further embodiments, the cyclooctene ester (also) comprises (a) cyclooctenyl arenecarboxylate. In yet further embodiments the cyclooctene ester (also) comprises (an) organyl cyclooctenecarboxylate. In further embodiments, the first compound (and the second compound) comprises one or more (substituted) cyclooctenes selected from the group consisting of (*E*/*Z*)-10,10-dimethyl-9,11-dioxabicyclo[6.3.0]undec-4-ene (see (*I*) in Fig. 3, depicting the respective *Z*-isomer), ethyl (*E*/*Z*)-bicyclo[6.1.0]non-4-ene-9-carboxylate (see (II) in Fig. 3 for the *Z*-isomer), ethyl 3-acetoxybicyclo[6.1.0]non-4-ene-9-carboxylate ((*III*) in Fig. 3 depicting the *Z*-siomer), (*E*/*Z*)-cyclooct-2-en-1-yl acetate ((*IV*) in Fig. 3 depicting the Z-isomer), (*E*/*Z*)-cyclooct-2-en-1,4-diyl diacetate ((*V*) in Fig. 3 depicting the *Z*-isomer) and (*E*/*Z*)-cyclooct-4-en-1-yl acetate ((*VI*) in Fig. 3, depicting the *Z*-isomer). Hence, in embodiments, the first compound compounds of the formula (*VII*), (*VIII*), (*IX*), and (*X*) depicted in Fig.3, (and wherein the second compound comprises an isomer of the first compound).

Especially, the first compound comprises one or more cyclooctenes selected from the group consisting of a diastereoisomer of (*E*)-cyclooct-4-en-1-yl acetate, a diastereoisomer of ethyl (1*R*,4*E*,8*S*,9*r*)-bicyclo[6.1.0]non-4-ene-9-carboxylate, a diastereoisomer of ethyl (1*R*,4*E*,8*S*,9*s*)-bicyclo[6.1.0]non-4-ene-9-carboxylate, and a diastereoisomer of (1*R*,4*E*,8*R*)-10,10-dimethyl-9,11-dioxabicyclo[6.3,0]undec-4-ene.

Especially, the *E*-isomer of these cyclooctenes may form a complex with the third compound, silver nitrate, which may dissolve in the aqueous liquid. Further, advantageously, the (corresponding) *Z*-isomer of these cyclooctenes may not form a complex with the third compound and may have a low solubility relative to said complex of the third compound and said *E*-isomer. In embodiments, a complex between *E*-cyclooctene and silver nitrate is formed, being the first and the third compound, respectively.

It was further surprisingly found that an addition of nitric acid to the second liquid (the aqueous liquid) comprising silver nitrate prevented a formation of an undesired precipitate (in the extraction unit). According to the invention, the second liquid, i.e. the aqueous liquid, comprises nitric acid, wherein the third compound is silver nitrate. aqueous liquid may be selected in the range of 0-7, especially in the range of 3-5. In embodiments, nitric acid is provided to the second liquid. In further embodiments, nitric acid is provided to the extraction unit.

The method may provide the first compound in the second liquid, especially in a complex formed between the first compound and the third compound. The method may further comprise an isolation stage. Said isolation stage may comprise isolating the first compound from the second liquid, especially after removing the second liquid from the extraction unit. In embodiments, the isolation stage further comprises disintegrating the complex formed between the third compound (such as a silver salt, especially silver nitrate) and the first compound (especially *E*-cyclooctene*)* and successively extracting the first compound in a second organic liquid. Especially, the isolation stage may further comprise (i) washing the second organic phase to provide a washed organic liquid, and especially (ii) drying the washed organic liquid, and optionally (iii) removing the second organic liquid, such as by evaporation, to provide the first compound either in dried form or in the organic phase. The second organic liquid may comprise the organic liquid (comprised by the first liquid). In other embodiments, the second organic liquid does not comprise the organic liquid (comprised by the first liquid). In embodiments, the complex is disintegrated by providing ammonium hydroxide to the second liquid after removing it from the extraction unit. The second organic liquid may e.g. comprise a hydrocarbon, such as one or more selected from the group consisting of pentane, hexane, heptane, benzene, toluene, xylene, etc.. In a second aspect, the invention provides a system for a continuous production of (a product comprising) a first compound, especially the first compound (and/or the product comprising the first compound) described herein. The system used according to the

invention is used for the production of the first compound using the method of the invention. Hence, (elements of) embodiments of the system may comprise (elements of) embodiments of the method and vice versa. Especially, the system comprises the extraction unit described herein. In further embodiments, the system further comprises an isomerization reactor, especially configured for an isomerization of an isomer. Especially, the isomerization reactor is configured to provide the isomerization stage of the method. Hence, the isomerization stage (of the invention) may take place in the isomerization reactor (of the invention).

Hence, the invention provides a use of a system for a continuous production of (a product comprising) a first compound, the system comprising (i) an extraction unit, (ii) a flow device configured to continuously remove at least part of a first liquid or a second liquid, especially the first liquid, from a first withdrawal zone of the extraction unit, and optionally (iii) an isomerization reactor comprising a reactor inlet (arranged) in fluid contact with the first withdrawal zone of the extraction unit, a reactor outlet (arranged) in fluid contact with a mixing zone of the extraction unit and a flow channel (arranged) in fluid contact with the reactor inlet and (arranged) (in fluid contact) with the reactor outlet. The flow channel is especially arranged between the reactor inlet and the reactor outlet.

The flow device is especially configured to provide a (circulating) flow of a liquid, especially the first liquid, from the first withdrawal zone of the extraction unit (especially via the isomerization reactor) (and especially through the flow channel) to the mixing zone of the extraction unit, and may for instance comprise a (liquid) pump. A flow device may further relate to more than one flow devices. Also a flow channel may further relate to more than one flow channel (all in fluid contact with the reactor inlet and with the reactor outlet). In embodiments, the flow device is arranged between the reactor outlet (of the isomerization reactor) and the mixing zone of the extraction unit. Yet, in further embodiments, the flow device is arranged between the first withdrawal zone of the extraction unit and the reactor inlet (of the isomerization reactor). The phrase "arranged between" "a first location" and "a second location" and the like especially relates to being configured in fluid (liquid) connection with the first location and (being configured) in fluid (liquid) connection with the second location. For instance, arranged between the reactor outlet and the mixing zone, especially relates to being configured in fluid (liquid) connection with the reactor outlet and (being configured) in fluid (liquid) connection with the mixing zone.

Especially, using the system used according to the invention may allow continuously providing the first compound and may easily be scaled-up. The system, especially the isomerization reactor, further allows continuously enriching the first liquid with the first compound, especially positively facilitating any extraction of the first compound from the first liquid in the extraction unit. Further, using the system may not require specific devices to liberate the first compound from the extraction unit.

The extraction unit may comprise an extraction unit volume selected in the range of 1 ml. - 1 1., especially 100 ml. - 1000 ml. In other embodiments the extraction volume is selected in the range of 1 1. - 1000 1., especially in the range of 1 1. - 100 1., more especially in the range of 1.5 1. - 10 1.. The mixing zone may comprise a mixing volume. Essentially, the mixing volume comprises at least a part of the extraction unit volume. Especially a ratio of the mixing volume to the extraction unit volume comprises at least 0.5, especially at least 0.6, such as selected in the range of 0.6 - 0.99, especially selected in the range of 0.7-0.9. In further embodiments, said ratio is smaller than 0.5. Especially said ratio is at least 0.05. The extraction unit volume further comprises a volume of the first withdrawal zone ("first withdrawal zone volume". The extraction volume may further comprise one or more of a volume of the (optional) second withdrawal zone ("second withdrawal volume") and a volume of the (optional) introduction zone ("introduction volume").

Hence the extraction unit comprises zones. Especially, the extraction unit comprises a mixing zone and a first withdrawal zone, and optionally at least one or more of a second withdrawal zone and an introduction zone. One or more of said zones may comprise discrete zones, especially being at least partly physically separated from another zone. Separation may for instance be provided by a membrane, e.g. to separate the organic phase from the aqueous phase, especially to separate a withdrawal zone from the mixing zone (see also below). In further embodiments, one or more zones are not physically separated (from one or more other zone(s)). Yet in further embodiments, one or more zones overlap. Especially the respective volumes of the zones may overlap. Especially at least one of such volume may (partly) comprise a volume of another zone. Especially, the first withdrawal zone and the second withdrawal zone do not overlap. Yet in further embodiments, the mixing zone comprises the introduction zone. Especially, the zones are in fluid contact with each other.

Especially the first withdrawal volume, and/or the (optional) second withdrawal volume, and/or the (optional) introduction volume is/are smaller than the mixing volume.

The extraction unit especially comprises a liquid-liquid extraction unit. The extraction unit, especially the mixing zone is configured to provide a liquid-liquid interface ("liquid interface" or "contact (area)" between the first liquid and the second liquid, especially to allow a transport of the first compound from the first liquid to the second liquid. Hence, the extraction unit, especially the extraction volume, may comprise a mixing means. At least one of the zones may comprise (at least part of) the mixing means. The mixing means may comprise mixing elements. Hence, especially one or more zones may comprise a mixing element. A mixing mean may further relate to more than one (different) mixing means.

Especially, the mixing zone comprises at least part of the mixing means (to increase said liquid interface). In embodiments, the mixing zone may comprise a dynamic mixer. The mixing zone may for instance comprise a stirrer or a magnetic stir bar ("flea"). In further embodiments, the mixing zone may additionally or alternatively comprise a static mixer. The mixing zone may also comprise a packing material, especially wherein the packing material is configured e.g. in a column. Especially such embodiment may comprise one or more discrete zones. For instance, the packing material may comprise the mixing zone and especially the first withdrawal zone may be configured at a location not comprising packing material.

In embodiments, the amount of first compound in the first liquid may be increased by the addition of second compound which may be (partly) isomerized to the first compound in the isomerization reactor. Hence, further embodiments of the system are configured for introduction of the second liquid, especially in the mixing zone. Especially the introduction zone is configured in fluid contact with the mixing zone of the extraction unit.

In embodiments, the system further comprises a second compound supply device (arranged) in fluid contact ("directly" or "via" the isomerization reactor) with the mixing zone of the extraction unit and configured to enrich the first liquid with a second compound. Especially, the second compound supply device may be configured to enrich the first liquid with the second compound at a location downstream of the extraction unit and upstream of the isomerization reactor. Alternatively (or additionally), the second compound supply device may be configured to enrich the first liquid with the second compound at a location upstream of the extraction unit and downstream of the isomerization reactor.

In embodiments, only the first liquid may continuously be withdrawn from the extraction unit, especially from the first withdrawal zone. Especially, the system is configured to circulate the first liquid, especially wherein the first liquid may be transported from the extraction unit through the isomerization reactor and to the mixing zone of the extraction unit. In further embodiments, the first liquid and the second liquid may be withdrawn from the extraction unit. Especially, the second liquid may be withdrawn from a second withdrawal zone of the extraction unit. Hence, in embodiments, the extraction unit further comprises a product withdrawal port configured for withdrawal of at least part of a (the) second liquid from the extraction unit, especially from a second withdrawal zone of the extraction unit. In further embodiments, the system is configured to add additional second liquid from a second liquid supply device to the system, especially to an introduction zone in the extraction unit. Hence, in further embodiments the extraction unit further comprises an introduction zone and the system further comprises a second liquid supply device arranged in fluid contact with the introduction zone of the extraction unit.

The extraction unit is especially configured to provide a separation of the withdrawal zone from the mixing zone and/or second withdrawal zone, especially such that only one of the first and second liquid may be removed (separately) from the first and/or the second withdrawal zone. The first liquid and the second liquid are especially immiscible. Especially, the first liquid and the second liquid may separate based on a difference in specific density. In embodiments, the extraction unit comprises a column, especially a packed column, wherein the first withdrawal zone and the mixing zone and/or the second withdrawal zone may be distanced from each other. In further embodiments the volume of the withdrawal zones are selected to be relatively large compared to the mixing volume. In yet further embodiments, the extraction unit further comprises a membrane, especially wherein the membrane is configured to separate the first liquid from the second liquid. In embodiments, the membrane comprises a hydrophilic membrane. In further embodiments, the membrane comprises a hydrophobic membrane. Especially, the membrane may be configured substantially only to be permeable by the organic liquid. Such membrane may for instance be arranged in the first and/or the second withdrawal zone, especially to separate the respective zones. In further embodiments a membrane is configured between the first withdrawal zone and the mixing zone.

The isomerization reactor, especially comprising a reactor volume, is essentially configured to allow isomerization of at least a part of the second compound (into the first compound). In embodiments, the reactor volume is selected in the range of 1 ml - 1 1, especially 100 ml. - 1000 ml. In other embodiments the reactor volume is selected in the range of 1 1. - 1000 1., especially in the range of 1 1. - 100 1., more especially in the range of 1.5 1. - 101. According to the invention, the isomerization reactor comprises a photo-isomerization reactor. Especially, the photo-isomerization reactor ("photo reactor") is configured to provide light to the first liquid flowing through the flow channel, especially to the second compound and/or a sensitizer in said first liquid. Especially, the light comprises UV light. In embodiments, the photo-isomerization reactor is configured to provide (UV-)light, especially comprising a wavelength selected in the range of 100-280 nm, such as in the range of 240-270 nm, especially in the range of 250 nm-260 nm, such as comprising substantially 254 nm. Hence especially, at least part of the flow channel is configured to allow providing (UV) light to the first liquid (in the flow channel). Especially at least part of a wall of the flow channel is transmissive for said (UV) light. In further embodiments, the photo-isomerization reactor is configured to provide visible (and or near IR) light comprising a wavelength selected in the range of 380-800 nm, even more especially in the range of 420-500 nm.

In embodiments, the flow channel, especially at least part of the wall of the flow channel, comprises a light transmissive material (transmissive for light having e.g. the above indicated wavelength(s)). Such materials are known in the art and may comprise e.g. one or more of glass, fluorinated propene, quartz, and glass. Hence, in embodiments, the flow channel, especially at least part of the wall of the flow channel comprises at least one material selected from

the group consisting of glass, quartz, vycor, corex, pyrex, fluorinated ethylene propene, and poly(tetrafluoroethylene). The flow channel may have a characteristic dimension, such as a diameter of the flow channel or a width of the flow channel, selected to provide the light to a major part of the first liquid in the flow channel. Especially a large characteristic dimension may not allow the light to penetrate in a center of the flow channel. Hence, in embodiments, the characteristic dimension (the width) of the flow channel is selected in the range of 1-50 mm, especially 1 - 25 mm, even more especially in the range of 1-10 mm, such as 1-5 mm.

In further embodiments, the photo-isomerization reactor comprises a (UV) lighting element. Especially at least part of the flow channel is arranged surrounding the lighting element. Alternatively or additionally the (UV) lighting element may be arranged at least partly surrounding the flow channel. The (UV) lighting element may for instance comprise an emitting wall and configured to emit said light towards the flow channel. The lighting element may further comprise a reflector configured to reflect (emitted) light in a direction of the flow channel. In yet further embodiments, the photo reactor comprises a tubing configured to emit the light, wherein the tubing is configured around at least part of the flow channel. Especially such tubing may comprise (UV) light emitting elements. Especially, the lighting element comprises a UV-lighting element and especially, the light comprises UV-light (as described above). In further embodiments, the photo reactor comprises a (UV) lighting element, wherein at least part of the flow channel is arranged surrounding the (UV) lighting element. A lighting element may further relate to more than one (different) lighting element. Hence, a lighting element may e.g. relate to (a combination of) a lighting element providing UV light and a lighting element providing visible light, or any other type of combinations of wavelengths.

In yet further embodiments, the system further comprises a cooling system configured to cool (at least part of) the first liquid in the system. The cooling system may especially be arranged to cool (at least part of) the first liquid at any location in the system. Especially, the cooling system is arranged at a location wherein heat is supplied to the first liquid or at a location downstream of said location (wherein the heat is supplied). In embodiments, the isomerization reactor comprises said cooling system.

In embodiments, the system further comprises a sensor system, especially configured to sense a parameter of the second liquid in the extraction unit. In further embodiments, the system comprises a control system configured to control the second compound supply device and/or the product withdrawal port and/or the flow device. In embodiments, a concentration of the first compound in the second liquid may be sensed. In further embodiments, e.g. an amount of free silver nitrate (not being complexed with the first compound) may be sensed. In yet further embodiments, a volume of the second liquid in the exaction unit may be sensed. Especially based on the sensed parameter a "fresh" second liquid (especially, not comprising the first compound yet) may be provided to the extractor unit (by the second liquid device). Alternatively or additionally based on the sensed parameter, an amount of second liquid (comprising the first compound) may be removed from the extractor unit, especially from the second withdrawal zone.

Hence, especially, the system may comprise (i) an extraction unit comprising a mixing zone configured to provide contacting, especially mixing, of a first liquid, especially an organic liquid, with a second liquid, especially an aqueous liquid, wherein the first liquid and the second liquid are not miscible, and a first withdrawal zone in fluid contact with the mixing zone and (ii) a flow device configured to continuously remove at least part of the first liquid or the second liquid, especially at least part of the first liquid, from the withdrawal zone, wherein the first liquid comprises the first compound and a second compound, wherein the first compound, optionally in combination with a third compound, has a higher solubility in the second liquid than the second compound optionally in combination with the third compound, wherein the first compound is and *E*-isomer, especially *E*-cyclooctene, and the second compound is a (corresponding) *Z*-isomer, especially a (corresponding) *Z*-cyclooctene,

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the

appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to an advantage.

The various aspects discussed in this patent can be combined in order to provide additional advantages. Furthermore, some of the features can form the basis for one or more divisional applications.

It is noted that herein for clarity reasons the terms "*E*-isomer" and the like are in general used in relation to the first compound, wherein the terms "*Z*-isomer" and the like are in general used in relation to the second compound. It will be understood that these terms as well as the terms "first" compound and "second" compound are only used to discriminate between two corresponding isomers and two compounds having different properties and these terms may be interchanged. In some embodiments, e.g., the second compound is an *E*-isomer, wherein the first compound is a (corresponding) *Z*-isomer.

BRIEF DESCRIPTION OF THE DRAWINGS

Embodiments of the invention will now be described, by way of example only, with reference to the accompanying schematic drawings in which corresponding reference symbols indicate corresponding parts, and in which:

Fig. 1 schematically depicts an embodiment of the system used according to the invention;
Fig. 2 schematically depicts another embodiment used according to the invention; and
Fig 3. schematically depicts some embodiments of isomers.

The drawings are not necessarily to scale.

DETAILED DESCRIPTION OF THE EMBODIMENTS

Figure 1 schematically depicts an embodiment of the system used according to the invention. The figure further depicts different aspects of the system and the method of the invention. The figure depicts a system 1 for a continuous production of a first compound 12, which is an *E*-cyclooctene. The depicted system 1 comprises an extraction unit 100, a flow device 400, and an isomerization reactor 200.

The extraction unit 100 used according to the invention comprises a mixing zone 110 and a first withdrawal zone 120. In the depicted embodiment, the extraction unit 100 further comprises a second withdrawal zone 130 and an introduction zone 140. The depicted isomerization reactor 200 comprises a reactor inlet 210 and a reactor outlet 220 and a flow channel 230 arranged between the reactor inlet 210 and the reactor outlet 220. Hence, the flow channel 230 is in liquid connection with the reactor inlet 210 and the reactor outlet 220. The reactor inlet 210 is especially configured in fluid contact with the first withdrawal zone 120 (of the extraction unit 100), and the reactor outlet 220 is especially configured in fluid contact with the mixing zone 110. In the figure also schematically the mixing volume 115, the first withdrawal volume 125, the second withdrawal zone 135, and the introduction volume 145 are depicted with phantom lines (because the line marking the separation between the mixing volume 115 and the first withdrawal liquid 125 corresponds to the location where the mixing first liquid 10 separates from the mixture of the first liquid 10 and the second liquid 20 that line is not in phantom). In the depicted figure the mixing volume 115 comprises the introduction volume 145, whereas the first withdrawal volume 125 and the second withdrawal volume 135 are separated from each other (by the mixing volume 115). It is further noted that the mixing volume 115 is larger than the other volumes 125,135,145.

In the depicted embodiment, a first liquid 10 may be circulated through the system 1 especially by means of the flow device 400. Especially, the first liquid 10 comprises a first compound 12 and a second compound 13 and is provided to the mixing zone 110 in the extraction unit 100, wherein also a second liquid 20 may be present optionally comprising a third compound 22. Especially the first liquid 10 and the second liquid 20 may not be miscible. Especially the first liquid 10 comprises an organic liquid 11 and the second liquid 20 comprises an aqueous liquid 21. The liquids 11,21 may be contacted in the extraction unit 100, especially in the mixing zone 110, wherein at least a part of the first compound 12 may translocate/transfer from the first liquid 10 to the second liquid 20, for instance by forming a complex with said optional third compound 22 present in the second liquid 20 that may contact the first compound 12 in the first liquid 10 when the first liquid 10 contacts the second liquid 20. The first compound 12 may in embodiments also transfer directly to the second liquid 20 by mass transfer, such as by diffusion. A contact between the first liquid 10 and the second liquid 20 (in the mixing zone 110) may be improved by mixing in the mixing zone 110. In the figure mixing may be provided by the mixer 150, especially a dynamic mixer 151. Alternatively or additionally mixing may also be provided

by (simply) providing a flow of the first liquid in/through the second liquid, such as by allowing the first liquid to flow/bubble through the second liquid (or vice versa).

By means of the flow device 400 a part of the first liquid may (continuously) be withdrawn/removed from the extraction unit 100, especially from the first withdrawal zone 120 and be transported to the isomerization reactor 200, in the figure embodied by a photo reactor 240, herein also indicated as "photo-isomerization reactor" 240. The first liquid 10 may be transported through the flow channel 230 comprising a wall 231 that at least partly is transmissive for (UV) light. The flow channel 230 may for instance comprise a (light) transmissive material such as glass, quartz, vycor, corex, pyrex, fluorinated ethylene propene or poly(tetrafluoroethylene). The photo-isomerization reactor 240 may especially be configured to provide UV light, such as comprising a wavelength selected in the range of 240-270 nm. Especially the UV light may be provided by an UV lighting device 250. In the depicted embodiment the flow channel 230 is arranged (at least partly) surrounding the lighting element 250. In further embodiments, the lighting device 250 may be arranged (partly) surrounding the flow channel 230. In other embodiments, the photo-isomerization reactor 240 may be configured to provide visible light, especially being provided by a lighting device providing visible light. In embodiments, also the flow channel 230 may be arranged (partly) surrounding the such lighting device or such lighting device may be arranged (partly) surrounding the flow channel 230.

In the isomerization reactor 200 a part of the second compound 13 may be isomerized / converted in the isomerization stage into the first compound 12. The light may also heat up the first liquid 10. Hence the invention also provides embodiments comprising a cooling system 300, especially configured to cool the first liquid 10 and/or the second liquid 20. In the depicted embodiment, the isomerization reactor 200 comprises said cooling system 300, such as a cooling jacket 310 arranged around the flow channel 230. Especially, the isomerization stage (of the method) may comprise cooling the first liquid 10. Especially, the cooling system 300 is arranged at a location upstream of the extraction unit (100).

In the isomerization reactor 200, the first liquid 10 may especially be enriched with said first compound 12 (by an isomerization of the second compound 13). After enriching the first liquid 10 with the first compound 12, the first liquid 10 may again be provided to (the mixing zone 110 in) the extraction unit 100, wherein again a part of the first compound 12 may transfer to the second liquid 20. Hence especially the first liquid 10 may comprise a circulating first liquid.

It is noted that the flow device 400 may be arranged as indicated in figure 1, i.e. downstream of the first withdrawal zone 120 and upstream of the isomerization reactor inlet 210. In other embodiments, the flow device 400 is arranged upstream of the mixing zone 110 of the extraction unit 100 and downstream of the reactor outlet 220.

The depicted embodiment further comprises a second compound supply device 99 that is also configured in fluid contact with the mixing zone 110 of the extraction unit 100. The second compound supply device 99 is configured to enrich the first liquid 10 with the second compound 13. In the depicted embodiment the supply device 99 is configured at a location upstream of the extraction unit 100 and downstream of the isomerization reactor 200. In other embodiments, the supply device 99 is configured downstream of the extraction unit 100 and upstream of the isomerization reactor 200. Hence the first liquid 10 may be enriched with the second compound 13 at a location upstream of an isomerization reactor 200, wherein said isomerization stage takes place, and downstream of said extraction unit 100 or at a location downstream of said isomerization reactor 200 and upstream of said extraction unit 100.

The depicted embodiment further comprises a product withdrawal port 170 configured in the extraction unit 100. Said port 170 is especially configured to allow removing at least part of a second liquid 20 from the extraction unit 100. Additionally, the depicted embodiment comprises a second liquid supply device 500 that is also configured in fluid contact with the mixing zone 110 of the extraction unit 100, via the introduction zone 140 of the extraction unit 100. Via the second liquid supply device 500 additional (fresh) second liquid 20, optionally comprising the third compound 22 may be introduced in the extraction unit 100. The withdrawal port 170 and/or the introduction zone 140 may in further embodiments be arranged at another location. Especially, the withdrawal port 170 is arranged at a location comprising substantially no first liquid. Especially, the introduction zone 140 may be arranged at a distance from the withdrawal port 170. The introduction zone may e.g. be arranged to supply the second liquid 20 at a location comprising mainly the first liquid 10, such as in or near the first withdrawal zone 120.

The system used according to the invention is used for performing embodiments of the method of the invention. The method, however, may also be performed without the isomerization reactor 200 and especially with or without a circulating first liquid 10. In the method the first liquid 10 comprising the first compound 12 and the second compound 13 is provided to the mixing zone 110 in the extraction unit 100, wherein the first liquid 10 is contacted / mixed with the second liquid 20 that is especially not miscible with the first liquid 10. In the first withdrawal zone 120 of the extraction unit 100 and in fluid contact with the mixing zone 110 at least part of the first liquid 10 may be removed. However, alternatively (also) at least part of the second liquid 20 may continuously be removed from said extraction unit 100, especially if "fresh" second liquid 20 is supplied via a second liquid supply device 500 such as is depicted in figure 1. In the method, the first liquid 10 may be provided as a circulating first liquid as is depicted in figure 1. Additionally or alternatively, the first liquid 10 may be provided by an extra introduction of a first liquid 10, e.g. by a first liquid supply device 95 (indicated by dashed lines). Especially this way a circulating first liquid 10 may be enriched with the first

compound 12 and the second compound 13. Especially if no circulating first liquid 10 is used, at least a part of the first compound 12 in the first liquid 10 provided by the first liquid supply device 95 may be extracted in the second liquid 20 in the extraction unit 100.

The method further provides that the first liquid 10 may be removed from the second withdrawal zone 130 in the extraction unit 100, wherein at least part of the second liquid 20 is continuously removed from the first withdrawal zone 120 of the extraction unit 100. In the in figure 1 depicted embodiment the first liquid 10 is (continuously) removed from the first withdrawal zone 120, wherein the second liquid 20 may be removed from the second withdrawal zone 130.

A transfer of the first compound 12 from the first liquid 10 in the mixing zone 110 may especially be based on a difference of solubility of the first compound 12 and the second compound 13 in the second liquid 20. Optionally, the solubility of the first compound 12 and/or second compound 13 may be changed by an interaction of the first compound 12 and/or second compound 13 with a third compound 22 in the second liquid 20. In embodiments, for instance the third compound 22 may comprise a metal salt 23. Especially when a first compound 12 meets/contacts the third compound 22 (at an interface between the first liquid 10 and the second liquid 20) a combination of the first compound 12 and the third compound 22 may be formed, that may have a higher solubility in the second liquid 20 than the second compound 13 alone or a combination of the second compound 13 with the third compound 22. In embodiments for instance complex 24 may be formed between a metal salt 23, especially silver nitrate and an *E*-cyclooctene (as an example of the first compound 12) that has a higher solubility in the second liquid 20 than *Z*-cyclooctene (being a corresponding second compound 13).

Especially in the method said first liquid 10 is an organic liquid 11, especially (n-) heptane, and said second liquid is an aqueous liquid 21.

In embodiments, the first liquid 10 further comprises a sensitizer 15 that may facilitate the photo-isomerization in the isomerization stage. The sensitizer 15 may for instance comprise acetophenone, benzophenone, benzil, acridine, duroquinone, pyrene, eosin or methyl benzoate

In figure 2, another embodiment of the system 1 is depicted, wherein the extraction unit 100 comprises a column. In such extraction unit 100 mixing may for instance be provided by a static mixer 150, 152, such as packing elements. In the figure, further a control system 600 is depicted that e.g. may control valves 610 and the flow device 400 based on sensors 620, such as a sensor 625 sensing e.g. a parameter of the second liquid 20 in the extraction unit 100. In further embodiments, the system comprises a control system configured to control the second compound supply device 500.

In Fig. 3 some embodiments of relevant *E* or *Z* isomers of olefins, relevant first or second compounds are depicted. Compound (*I*) schematically depicts (*Z*)-10,10-dimethyl-9,11-dioxabicyclo[6.3.0]undec-4-ene, (*II*) depicts ethyl (*Z*)-bicyclo[6.1.0]non-4-ene-9-carboxylate, (*III*) depicts (the *Z* isomer of) ethyl 3-acetoxybicyclo[6.1.0]non-4-ene-9-carboxylate, (*IV*) depicts (*Z*)-cyclooct-2-en-1-yl acetate, (*V*) depicts (*Z*)-cyclooct-2-en-1,4-diyl diacetate, and (*VI*) depicts (*Z*)-cyclooct-4-en-1-yl acetate.

Especially, (VII) - (X) depict embodiments of the first compound 12 of the invention, wherein (*VII*) depicts both diastereoisomers of (*E*)-cyclooct-4-en-1-yl acetate, (*VIII*) the diastereoisomers of ethyl (1*R*,4*E*,8*S*,9*r*)-bicyclo[6.1.0]non-4-ene-9-carboxylate, (*IX*) diastereoisomers of ethyl (1*R*,4*E*,8*S*,9*s*)-bicyclo[6.1.0]non-4-ene-9-carboxylate, and (*X*) diastereoisomers of (1*R*,4*E*,8*R*)-10,10-dimethyl-9,11-dioxabicyclo[6.3.0]undec-4-ene.

Embodiments (*XI*) - (*XIII*) schematically depict the general structure of the first compound 12 of the invention. The structure of the cyclooctene comprises a cyclooctene ester or a protected cyclooctenediol. The cyclooctene ring comprises one double bound, having further a respective hydrogen atom bound to each of the two carbons (carbon atoms) joined to the double bound Functional groups may be bound to the remaining 6 C-atoms in the ring. These functional groups are depicted by X and/or by OR. The functional group may also comprise a cyclic functional group, such as indicated in the compounds depicted by formulas (*I*), (*II*), (*III*), (*VIII*), (*IX*), (*X*), (*XIV*), (*XV*), and (*XVI*). The compound depicted by formulas (*I*) and (X) exemplify Z-isomer and *E*-isomers, respectively of embodiments depicted by formula *(XIII)* wherein R is ethyl, and wherein X is linked to a carbon (atom) of the -OR group forming the cyclic group (comprising $(O)_2$-C-$(methyl)_2$). The compounds depicted in formula (*XIV*), (*XV*) and (*XVI*) comprise a (substituted) cyclopropyl group. Especially said cyclopropyl group may comprise an ester, see the compound depicted by formula (*VIII*) and formula (*IX*).

The "undulated" bond, also referred to as wiggled bond, relates to a substituent that may be arranged at either sides of the ring structure, especially indicating (two) different diastereoisomers.

It is noted that especially the spatial orientation of the functional group with respect to the ring structure may not depicted in the figure (at all), to schematically depict a mixture of diastereoisomers.

EXPERIMENTAL

Experiments were performed in a system comparable with the one depicted in Figure 1. The photoreactor consited out of fluorinated ethylene propylenen (FEP) tubing (ID 2.7 mm) coiled around a Philips PL-L 55W UV lamp (λ

= 254 nm). The tubing was externally cooled with water. The extraction unit was a customized round-bottom flask (100 mL) with an extended neck and a magnetic stirrer. Phase seperation is taking place in the modified flask. The exposure time could be regulated with the flow rate of the pump.

The organic liquid was circulated by the pump, and the aqueous liquid was operated batch wise (initially provided to the extractraction unit and only removed from the extraction unit when the experiment was stopped). A starting concentration of the substrate (ZCO) was set at the beginning of the experiment. The concentration was maintained by adding substrate to the system after the UV exposure.

For the different substrates (isomers), refence in made to Fig. 3 for the respective structural formulas.

### General procedure for isomerization

The tubing of the setup (total volume: 105 mL, residence volume: 100 mL) was rinsed with degassed n-heptane. Then, it was filled with a solution of substrate (15.5 mmol) and methyl benzoate (3.1 mmol, 0.2 equiv) (sensitizer) in degassed n-heptane (100 mL). A solution of silver nitrate (70 mmol, 4.5 equiv) in a 0.1 mM nitric acid solution in water (140 mL) with degassed n-heptane (30 mL) was added to the 100 mL extraction unit such that the extended neck was partially filled. The pump inlet tube was placed at the top of the organic phase (for removing the organic liquid from the extraction unit), the reactor output tube (for introducing the organic liquid in the flask) was placed just above the stirring magnet in the aqueous phase. The organic phase was pumped through the entire system for about 5 cycles without exposure (to UVlight) while argon bubbled through the extration unit to remove remaining oxygen left in the reaction mixture. The exposure time ranged from 5 to 20 minutes for several experiments and was done at a wavelength of 254 nm. To maintain the substrate concentration in the organic phase, pure substrate was added to the system by a syringe pump after exposure and before entering the extration unit. The addition speed was determined by the estimated generation of ECO: $Y_{ECO}$ (mg/min) calculated with the equation $Y_{ECO} = \frac{Q_{ECO} \cdot C_{ZCO} \cdot v_{flow}}{M_w}$, wherein$Q_{ECO}$ = ratio of ECO conversion, $C_{ZCO}$ = concentration of substrate at start (mmol/mL), $v_{flow}$ = flow rate (mL/min), and $M_w$ = molecular weight (mg/mmol). The setup ran between 1 and 6 hours in several experiments.

### General procedure for product extraction

After stopping the isomerization, the silver nitrate (1 equiv) aqueous phase is separated with a separatory funnel and washed with two portions of n-heptane (2 × 25 vol.%). n-Heptane (50 vol.%) and ammonium hydroxide (4 equiv) are added to the aqueous phase to extract the product. The obtained organic phase is washed with water (2 × 25 vol.%), dried with sodium sulfate and concentrated in vacuo to afford the product (ECO) with little impurities.

### Synthesis of (*E*)-cyclooct-4-en-1-yl acetate (*E*)-*VII*a/(*E*)-*VII*b

According to the general procedure, compound **(*Z*)-*VII*** (3.0 g, 17.83 mmol) was isomerized with 5 minutes exposure time to afford a mixture of both diastereoisomers **(*E*)-*VII*a** and (*E*)-***VII*b** (63:37, 2.06 g, 12.22 mmol) (see (*VII*) in Fig. 3) as a mixture in 1 hour with substrate addition (2.8 g, 16.64 mmol). $^1$H NMR (500 MHz, $CDCl_3$) δ 5.62-5.39 (m, 2 × 2H), 4.98-4.89 **((*E*)-*VII*b,** m, 1H), 4.42-4.33 **((*E*)-*VII*a,** m, 1H), 2.37-2.00 (m, 6H), 2.04 **((*E*)-*VII*b,** s, 3H), 1.93 **((*E*)-*VII*a,** s, 3H), 1.97-1.45 (m, 6H), 1.44-1.33 (m, 1H), 1.22-1.11 (m, 1H). $^{13}$C NMR (126 MHz, $CDCl_3$) δ 170.21, 170.11, 135.24, 134.86, 132.99, 131.63, 80.01, 69.95, 40.89, 40.73, 38.61, 34.27, 34.25, 32.52, 32.19, 30.97, 29.91, 28.17, 21.45, 21.06.

### Synthesis of ethyl (1*R*,4*E*,8*S*,9*r*)-bicyclo[6.1.0]non-4-ene-9-carboxylate (*E*)-*VIII*

According to the general procedure, compound **(*Z*)-*VIII*** (3 g, 15.44 mmol) was isomerized with 20 minutes exposure time to afford compound **(*E*)-*VIII*** (5.75 g, 29.59 mmol) (see (*VIII*) in Fig. 3) as a mixture of diastereoismers in 5.5 hours with substrate addition (6.0 g, 30.88 mmol). $^1$H NMR (500 MHz, $CDCl_3$) δ 5.85 (ddd, *J* = 16.8, 9.4, 6.2 Hz, 1H), 5.12 (dddd, *J* = 16.9, 10.6, 3.9, 1.2 Hz, 1H), 4.07 (q, *J* = 7.2 Hz, 2H), 2.41-2.33 (m, 1H), 2.31-2.16 (m, 3H), 2.00-1.87 (m, 2H), 1.29-1.20 (m, 1H), 1.22 (t, *J* = 7.2 Hz, 3H), 1.17-1.08 (m, 1H), 0.90 (t, *J* = 5.2 Hz, 1H), 0.95-0.81 (m, 1H), 0.59 (dddd, *J* = 14.1, 12.8, 11.7, 2.5 Hz, 1H). $^{13}$C NMR (126 MHz, $CDCl_3$) δ 174.78, 138.14, 131.62, 60.44, 38.06, 33.40, 32.01, 27.38, 27.32, 26.97, 25.89, 14.38.

### Synthesis of ethyl (1*R*,4*E*,8*S*,9*s*)-bicyclo[6.1.0]non-4-ene-9-carboxylate (*E*)-*IX*

According to the general procedure, compound **(*Z*)-*IX*** (1000 mg, 5.15 mmol) was isomerized with 20 minutes exposure time to afford compound **(*E*)-*IX*** (1100 mg, 5.67 mmol) (see (*IX*) in Fig. 3) as a mixture of diastereoisomers in

24 hours with substrate addition (1400 mg, 7.22 mmol). $^1$H NMR (400 MHz, $CDCl_3$) δ 5.80-5.68 (m, 1H), 5.29-5.17 (m, 1H), 4.02 (q, *J* = 7.1, 0.7 Hz, 2H), 2.38-2.23 (m, 2H), 1.99-1.81 (m, 4H), 1.76 (t, *J*= 9.0, 0.7 Hz, 1H), 1.73-1.61 (m, 1H), 1.51-1.37 (m, 1H), 1.19 (td, *J* = 7.1, 0.7 Hz, 3H), 1.15-1.04 (m, 1H), 1.04-0.91 (m, 1H). $^{13}$C NMR (101 MHz, $CDCl_3$) δ 172.02, 137.68, 132.26, 59.86, 33.58, 33.07, 27.08, 25.90, 24.45, 23.47, 20.95, 14.44.

**Synthesis of (1*R*,4*E*,8*R*)-10,10-dimethyl-9,11-dioxabicyclo[6.3.0]undec-4-ene (*E*)-*Xa*/(*E*)-*Xb***

According to the general procedure, compound **(*Z*)-*X*** (0.940 mg, 5.15 mmol) was isomerized with 20 minutes exposure time to afford a mixture of diastereoisomers **(*E*)-*X*a** and **(*E*)-*X*b** (78:22, 1360 mg, 7.47 mmol) (see (*X*) in Fig. 3) as a mixture of diastereoisomers in 16.5 hours with substrate addition (2275 mg, 12.5 mmol). The major isomer was separated by column chromatography (3% EtOAc in n-heptane): $R_F$: 0.18 and $R_F$: 0.24.

*Mixture:* $^1$H NMR (400 MHz, $CDCl_3$) δ 5.80 (t, *J* = 3.9 Hz, 2H), 5.45-5.30 (m, 2H), 3.72-3.61 (m, 2H), 3.64-3.54 (m, 2H), 2.47-2.33 (m, 2H), 2.31-1.98 (m, 10H), 1.94-1.80 (m, 2H), 1.77-1.61 (m, 2H), 1.28 (s, 6H), 1.27 (s, 6H).
**(*E*)-*X*a:** $^1$H NMR (500 MHz, $CDCl_3$) δ 5.52-5.35 (m, 2H), 3.80-3.66 (m, 2H), 2.36-2.27 (m, 2H), 2.29-2.16 (m, 4H), 1.82-1.64 (m, 2H), 1.33 (s, 6H). $^{13}$C NMR (126 MHz, $CDCl_3$) δ 133.03, 106.43, 84.45, 38.16, 31.58, 26.56.

## Claims

1. A method for a continuous production of a first compound (12), the method comprising continuously providing a first liquid (10) to a mixing zone (110) in an extraction unit (100), wherein the first liquid (10) comprises the first compound (12) and a second compound (13), mixing in the mixing zone (110) the first liquid (10) with a second liquid (20), wherein the first liquid (10) and the second liquid (20) are not miscible, wherein the first compound (12), in combination with a third compound (22), has a higher solubility in the second liquid (20) than the second compound (13) in combination with the third compound (22), wherein in a first withdrawal zone (120) in the extraction unit (100), in fluid contact with the mixing zone (110), at least part of the first liquid (10) is continuously removed from the extraction unit (100), wherein the first compound (12) is an *E*-cyclooctene, wherein the second compound (13) is a Z-cyclooctene, wherein the first liquid (10) is an organic liquid (11), wherein the second liquid (20) is an aqueous liquid (21), and wherein the third compound (22) is a metal salt (23), wherein

- the first compound (12) is a compound selected from the group consisting of compounds of the formula (XI), (XII), (XIII), (XIV), (XV), and (XVI),

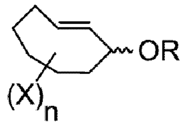
*(XI)*

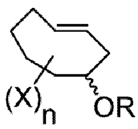
*(XII)*

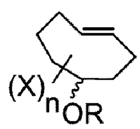
*(XIII)*

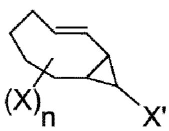
*(XIV)*

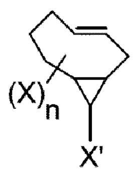
*(XV)*

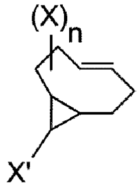
*(XVI)*

,

comprising a cyclooctene ring comprising two carbons joined to a double bound, wherein:

- X and X' are independently selected from the group consisting of hydrogen, halogen, -OR, $-C(O)_2R^2$, $-NO_2$, -CN, $-S(O)_2R$, $C_1$ - $C_{12}$ alkyl groups, $C_1$ - $C_{12}$ aryl groups, $C_1$ - $C_{12}$ alkylaryl groups, and $C_1$ - $C_{12}$ arylalkyl groups, wherein the alkyl groups, aryl groups, alkylaryl groups and arylalkyl groups are optionally substituted, and wherein R is independently selected from the group consisting of hydrogen, halogen, $C_1$ - $C_{24}$ alkyl groups, $C_6$ - $C_{24}$ (hetero)aryl groups, $C_7$ - $C_{24}$ alkyl(hetero)aryl groups and $C_7$ - $C_{24}$ (hetero)arylalkyl groups, wherein the alkyl groups, (hetero)aryl groups, alkyl(hetero)aryl groups and (hetero)arylalkyl groups are independently optionally substituted with one or more substituents independently selected from

the group consisting of $C_1$ - $C_{12}$ alkyl groupa, $C_2$ - $C_{12}$ alkenyl groups, $C_2$ - $C_{12}$ alkynyl groups, $C_3$ - $C_{12}$ cycloalkyl groups, $C_1$ - $C_{12}$ alkoxy groups, $C_2$ - $C_{12}$ alkenyloxy groups, $C_2$ - $C_{12}$ alkynyloxy groups, $C_3$ - $C_{12}$ cycloalkyloxy groups, halogens, amino groups, oxo groups, and silyl grpoups, wherein the silyl groups are represented by the formula $(R^3)_3Si$-, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S, and wherein $R^3$ is independently selected from the group consisting of $C_1$ - $C_{12}$ alkyl groups, $C_2$ - $C_{12}$ alkenyl groups, $C_2$

- $C_{12}$ alkynyl groups, $C_3$ - $C_{12}$ cycloalkyl groups, $C_1$ - $C_{12}$ alkoxy groups, $C_2$ - $C_{12}$ alkenyloxy groups, $C_2$ - $C_{12}$ alkynyloxy groups and $C_3$ - $C_{12}$ cycloalkyloxy groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups optionally being interrupted by one of more hetero-atoms selected from the group consisting of O, N and S, and $R^2$ is independently selected from the group consisting of $C_1$ - $C_{24}$ alkyl groups, $C_6$ - $C_{24}$ (hetero)aryl groups, $C_7$ - $C_{24}$ alkyl(hetero)aryl groups, and $C_7$ - $C_{24}$ (hetero)arylalkyl groups, wherein the alkyl groups, (hetero)aryl groups, alkyl(hetero)aryl groups and (hetero)arylalkyl groups are optionally substituted; wherein X can be located at any position on the cyclooctene ring other than at the two carbons joined to the double bound, and wherein X is optionally linked to a carbon atom in the -OR group, forming a cyclic group; and

- (i) n is at maximum 13, wherein the first compound (12) is a compound selected from the group consisting of compounds of the formula (XI), (XII), (XIII), or (ii) n is at maximum 12, wherein the first compound (12) is a compound selected from the group consisting of compounds of the formula (XIV), (XV), and (XVI), and wherein

- the second compound (13) comprises an isomer of the first compound (12),
- the third compound (22) is silver nitrate,
- the second liquid (20) comprises the third compound (22) and an aqueous nitric acid solution, and

wherein the method further comprises reintroducing the first liquid (10) to the mixing zone (110) after subjection of the first liquid (10) to an isomerization stage wherein the first liquid (10) is enriched with the first compound (12), wherein isomerization in the isomerization stage comprises photo-isomerization.

**2.** The method according to claim 1, wherein first compound (12) is a compound selected from the group consisting of a cyclooctene ester and a protected cyclooctenediol.

**3.** The method according to claim 2, wherein the cyclooctene ester comprises a cyclooctenyl alkanoate and/or an organyl cyclooctenecarboxylate.

**4.** The method according to any of the preceding claims, wherein in a second withdrawal zone (130) in the extraction unit (100), in fluid contact with the mixing zone (110), at least part of the second liquid (20) is removed from the extraction unit (100), the method further comprising: providing the second liquid (20) to the extraction unit (100) in an introduction zone (140) of the extraction unit (100), wherein the introduction zone (140) is in fluid contact with the mixing zone (110).

**5.** The method according to any of the preceding claims, wherein the isomerization stage comprises subjecting the first liquid to UV light, external from the extraction unit (100).

**6.** The method according to any of the preceding claims, wherein the first liquid (10) further comprises a sensitizer (15) selected from the group consisting of acetophenone, benzophenone, benzil, acridine, duroquinone, pyrene, eosin, and methyl benzoate.

**7.** The method according to any of the preceding claims, wherein the first liquid (10) is enriched with the second compound (13) at a location downstream of an isomerization reactor (200) wherein said isomerization stage takes place, and upstream of said extraction unit (100).

**8.** The method according to any of the preceding claims, wherein the first compound (12) comprises one or more cyclooctenes selected from the group consisting of 10,10-dimethyl-9,11-dioxabicyclo[6.3.0]undec-ene, ethyl-bicyclo[6.1.0]non-ene-9-carboxylate, ethyl-acetoxybicyclo[6.1.0]non-ene-9-carboxylate, cyclooctenyl acetate, and cyclooctenyl diacetate.

**9.** The method according to any of the preceding claims, wherein the first compound (12) comprises one or more cyclooctenes selected from the group consisting of compounds of the formula (*VII*), (*VIII*), (*IX*), and (*X*):

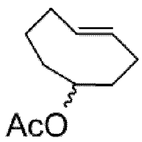

*(VII)*

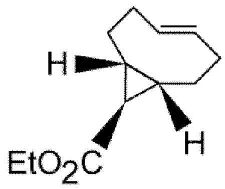

*(VIII)*

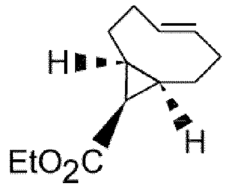

*(IX)*

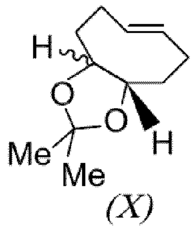

*(X)*

**10.** The method according to any of the preceding claims wherein a pH of the aqueous liquid is selected in the range of 3-5.

**11.** Use of a system (1) for carrying out the method of claim 1 for a continuous production of a first compound (12) as defined in claim 1, the system (1) comprising (i) an extraction unit (100), (ii) a flow device (400) configured to continuously remove at least part of a first liquid (10) from a first withdrawal zone (120) of the extraction unit (100), and (iii) an isomerization reactor (200) comprising a reactor inlet (210) in fluid contact with the first withdrawal zone (120) of the extraction unit (100), a reactor outlet (220) in fluid contact with a mixing zone (110) of the extraction unit (100) and a flow channel (230) in fluid contact with the reactor inlet (210) and in fluid contact with the reactor outlet (220), wherein

- the flow device (400) is configured to provide a circulating flow of the first liquid (10) from the first withdrawal zone (120) of the extraction unit (100) via the isomerization reactor (200) through the flow channel (230) to the mixing zone (110) of the extraction unit (100),
- the isomerization reactor (200) comprises a photo-isomerization reactor,
- the extraction unit (100) comprises a liquid-liquid extraction unit, configured for contacting the first liquid (10) with a second liquid (20), wherein the second liquid (20) comprises an aqueous nitric acid solution and silver nitrate and wherein the first liquid (10) and the second liquid (20) are immiscible, and
- the extraction unit (100) comprises mixing means.

**12.** The use of the system (1) according to claim 11, wherein the system (1) further comprises (iv) a second compound supply device (99) in fluid contact with the mixing zone (110) of the extraction unit (100) and configured to enrich the first liquid (10) with a second compound (13) at a location upstream of the extraction unit (100) and downstream of the isomerization reactor (200).

**13.** The use of the system (1) according to any of the claims 11-12, wherein the extraction unit (100) further comprises a product withdrawal port (170) configured for withdrawal of at least part of the second liquid (20) from the extraction unit (100), the extraction unit (100) further comprising an introduction zone (140), wherein the system (1) further comprises a second liquid supply device (500) arranged in fluid contact with said introduction zone (140) of the extraction unit (100).

**14.** The use of the system (1) according to any of the preceding claims 11-13, wherein the system (1) further comprises a cooling system (300) configured to cool at least part of the first liquid (10) in the system (1).

**15.** The use of the system (1) according to any of the preceding claims 11-14, wherein the photo-isomerization reactor

is configured to provide UV light comprising a wavelength selected in the range of 240-270 nm, wherein at least part of a wall (231) of the flow channel (230) is transmissive for said UV light, wherein the photo-isomerization reactor comprises a UV lighting element (250) configured to provide said UV light, wherein at least part of the flow channel (230) is arranged surrounding the lighting element (250).

**Patentansprüche**

1. Verfahren für eine kontinuierliche Herstellung einer ersten Verbindung (12), wobei man bei dem Verfahren in einer Extraktionseinheit (100) eine erste Flüssigkeit (10) einer Mischzone (110) zuführt, wobei die erste Flüssigkeit (10) die erste Verbindung (12) und eine zweite Verbindung (13) umfasst, in der Mischzone (110) die erste Flüssigkeit (10) mit einer zweiten Flüssigkeit (20) mischt, wobei die erste Flüssigkeit (10) und die zweite Flüssigkeit (20) nicht miteinander mischbar sind, wobei die erste Verbindung (12) in Kombination mit einer dritten Verbindung (22) in der zweiten Flüssigkeit (20) eine höhere Löslichkeit als die zweite Verbindung (13) in Kombination mit der dritten Verbindung (22) aufweist, wobei der Extraktionseinheit (100) an einer ersten Entnahmezone (120) in der Extraktionseinheit (100), die in Fluidkontakt mit der Mischzone (110) steht, zumindest ein Teil der ersten Flüssigkeit (10) kontinuierlich entnommen wird, wobei es sich bei der ersten Verbindung (12) um ein *E*-Cycloocten handelt, wobei es sich bei der zweiten Verbindung (13) um ein Z-Cycloocten handelt, wobei es sich bei der ersten Flüssigkeit (10) um eine organische Flüssigkeit (11) handelt, wobei es sich bei der zweiten Flüssigkeit (20) um eine wässrige Flüssigkeit (21) handelt und wobei es sich bei der dritten Verbindung (22) um ein Metallsalz (23) handelt, wobei

   - es sich bei der ersten Verbindung (12) um eine Verbindung handelt, die aus der Gruppe bestehend aus Verbindungen der Formeln (XI), (XII), (XIII), (XIV), (XV) und (XVI)

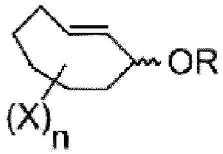
*(XI)*

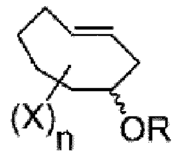
*(XII)*

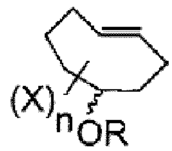
*(XIII)*

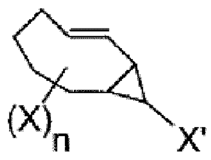
*(XIV)*

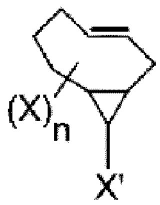
*(XV)*

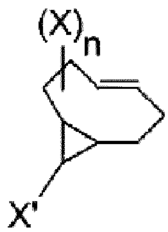
*(XVI)*

   ausgewählt ist, die einen Cyclooctenring, der zwei über eine Doppelbindung miteinander verbundene Kohlenstoffe umfasst, umfassen, wobei:

   - X und X' unabhängig aus der Gruppe bestehend aus Wasserstoff, Halogen, -OR, $-C(O)_2R^2$, $-NO_2$, -CN, $-S(O)_2R$, $C_1$-$C_{12}$-Alkylgruppen, $C_1$-$C_{12}$-Arylgruppen, $C_1$-$C_{12}$-Alkylarylgruppen und $C_1$-$C_{12}$-Arylalkylgruppen ausgewählt sind, wobei die Alkylgruppen, Arylgruppen, Alkylarylgruppen und Arylalkylgruppen gegebenenfalls substituiert sind und wobei R unabhängig aus der Gruppe bestehend aus Wasserstoff, Halogen, $C_1$-$C_{24}$-Alkylgruppen, $C_6$-$C_{24}$-(Hetero)arylgruppen, $C_7$-$C_{24}$-Alkyl (hetero) arylgruppen und $C_7$-$C_{24}$-(Hetero)arylalkylgruppen ausgewählt ist, wobei die Alkylgruppen, (Hetero)arylgruppen, Alkyl(hetero)arylgruppen und (Hetero)arylalkylgruppen gegebenenfalls unabhängig durch einen oder mehrere unabhängig aus der Gruppe bestehend aus $C_1$-$C_{12}$-Alkylgruppen, $C_2$-$C_{12}$-Alkenylgruppen, $C_2$-$C_{12}$-Alkinylgruppen, $C_3$-$C_{12}$-Cycloalkylgruppen, $C_1$-$C_{12}$-Alkoxygruppen, $C_2$-$C_{12}$-Alkenyloxygruppen, $C_2$-$C_{12}$-Alkinyloxygruppen, $C_3$-$C_{12}$-Cycloalkyloxygruppen, Halogenen, Aminogruppen, Oxogruppen und Silylgruppen ausgewählte Substituenten substituiert sind, wobei die Silylgruppen durch die Formel
   $(R^3)_3Si$- wiedergegeben werden, wobei die Alkylgruppen, Alkenylgruppen, Alkinylgruppen, Cycloalkylgruppen, Alkoxygruppen, Alkenyloxygruppen, Alkinyloxygruppen und Cycloalkyloxygruppen gegebenenfalls substituiert

sind, die Alkylgruppen, die Alkoxygruppen, die Cycloalkylgruppen und die Cycloalkoxygruppen gegebenenfalls durch ein oder mehrere aus der Gruppe bestehend aus O, N und S ausgewählte Heteroatome unterbrochen sind und wobei $R^3$ unabhängig aus der Gruppe bestehend aus $C_1$-$C_{12}$-Alkylgruppen, $C_2$-$C_{12}$-Alkenylgruppen, $C_2$-$C_{12}$-Alkinylgruppen, $C_3$-$C_{12}$-Cycloalkylgruppen, $C_1$-$C_{12}$-Alkoxygruppen, $C_2$-$C_{12}$-Alkenyloxygruppen, $C_2$-$C_{12}$-Alkinyloxygruppen und $C_3$-$C_{12}$-Cycloalkyloxygruppen ausgewählt ist, wobei die Alkylgruppen, Alkenylgruppen, Alkinylgruppen, Cycloalkylgruppen, Alkoxygruppen, Alkenyloxygruppen, Alkinyloxygruppen und Cycloalkyloxygruppen gegebenenfalls substituiert sind, die Alkylgruppen, die Alkoxygruppen, die Cycloalkylgruppen und die Cycloalkoxygruppen gegebenenfalls durch ein oder mehrere aus der Gruppe bestehend aus O, N und S ausgewählte Heteroatome unterbrochen sind und $R^2$ unabhängig aus der Gruppe bestehend aus $C_1$-$C_{24}$-Alkylgruppen, $C_6$-$C_{24}$-(Hetero)arylgruppen, $C_7$-$C_{24}$-Alkyl(hetero)arylgruppen und $C_7$-$C_{24}$-(Hetero)arylalkylgruppen ausgewählt ist, wobei die Alkylgruppen, (Hetero)arylgruppen, Alkyl(hetero)arylgruppen und (Hetero)arylalkylgruppen gegebenenfalls substituiert sind; wobei X sich an jeder beliebigen Position des Cyclooctenrings außer an den zwei über die Doppelbindung miteinander verbundenen Kohlenstoffen befinden kann und wobei X unter Bildung einer cyclischen Gruppe gegebenenfalls an ein Kohlenstoffatom der -OR-Gruppe gebunden ist, und

- (i) n höchstens 13 beträgt, wobei es sich bei der ersten Verbindung (12) um eine Verbindung handelt, die aus der Gruppe bestehend aus Verbindungen der Formeln (XI), (XII), (XIII) ausgewählt ist, oder (ii) n höchstens 12 beträgt, wobei es sich bei der ersten Verbindung (12) um eine Verbindung handelt, die aus der Gruppe bestehend aus Verbindungen der Formeln (XIV), (XV) und (XVI) ausgewählt ist, und wobei
- die zweite Verbindung (13) ein Isomer der ersten Verbindung (12) umfasst,
- es sich bei der dritten Verbindung (22) um Silbernitrat handelt,
- die zweite Flüssigkeit (20) die dritte Verbindung (22) und eine wässrige Lösung von Salpetersäure umfasst und wobei man bei dem Verfahren ferner, nachdem man die erste Flüssigkeit (10) einer Isomerisierungsstufe unterworfen hat, die erste Flüssigkeit (10) erneut in die Mischzone (110) einbringt, wobei die erste Flüssigkeit (10) mit der ersten Verbindung (12) angereichert wird, wobei die Isomerisierung bei der Isomerisierungsstufe Photoisomerisierung umfasst.

**2.** Verfahren nach Anspruch 1, wobei es sich bei der ersten Verbindung (12) um eine Verbindung handelt, die aus der Gruppe bestehend aus Cyclooctenester und einem geschützten Cyclooctendiol ausgewählt ist.

**3.** Verfahren nach Anspruch 2, wobei der Cyclooctenester ein Cyclooctenylalkanoat und/oder einen Organyl-Carbonsäurecyclooctencester umfasst.

**4.** Verfahren nach einem der vorhergehenden Ansprüche, wobei der Extraktionseinheit (100) an einer zweiten Entnahmezone (130) in der Extraktionseinheit (100), die in Fluidkontakt mit der Mischzone (110) steht, zumindest ein Teil der zweiten Flüssigkeit (20) entnommen wird, wobei man bei dem Verfahren ferner: der Extraktionseinheit (100) an einer Zuführzone (140) der Extraktionseinheit (100) die zweite Flüssigkeit (20) zuführt, wobei die Zuführzone (140) in Fluidkontakt mit der Mischzone (110) steht.

**5.** Verfahren nach einem der vorhergehenden Ansprüche, wobei man bei der Isomerisierungsstufe die erste Flüssigkeit außerhalb der Extraktionseinheit (100) UV-Licht unterwirft.

**6.** Verfahren nach einem der vorhergehenden Ansprüche, wobei die erste Flüssigkeit (10) ferner einen Sensibilisator (15) umfasst, der aus der Gruppe bestehend aus Acetophenon, Benzophenon, Benzil, Acridin, Durochinon, Pyren, Eosin und Methylbenzoat ausgewählt ist.

**7.** Verfahren nach einem der vorhergehenden Ansprüche, wobei die erste Flüssigkeit (10) an einer einem Isomerisierungsreaktor (200), wo die Isomerisierungsstufe stattfindet, nachgeschalteten und einer der Extraktionseinheit (100) vorgeschalteten Stelle mit der zweiten Verbindung (13) angereichert wird.

**8.** Verfahren nach einem der vorhergehenden Ansprüche, wobei die erste Verbindung (12) ein oder mehrere Cyclooctene umfasst, die aus der Gruppe bestehend aus 10,10-Dimethyl-9,11-dioxabicyclo[6.3.0]undecen, Ethylbicyclo[6.1.0]nonen-9-carboxylat, Ethylacetoxybicyclo[6.1.0]nonen-9-carboxylat, Cyclooctenylacetat und Cyclooctenyldiacetat ausgewählt sind.

**9.** Verfahren nach einem der vorhergehenden Ansprüche, wobei die erste Verbindung (12) ein oder mehrere Cyclooctene umfasst, die aus der Gruppe bestehend aus Verbindungen der Formeln (*VII*), (*VIII*), (*IX*) und (*X*):

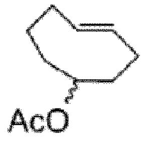

(VII)

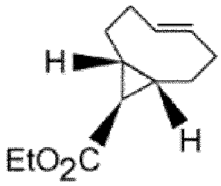

(VIII)

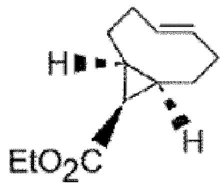

(IX)

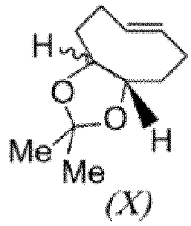

(X)

ausgewählt sind.

**10.** Verfahren nach einem der vorhergehenden Ansprüche, wobei ein pH-Wert der wässrigen Flüssigkeit aus dem Bereich 3-5 ausgewählt ist.

**11.** Verwendung eines Systems (1) zur Durchführung des Verfahrens nach Anspruch 1 für eine kontinuierliche Herstellung einer wie in Anspruch 1 definierten ersten Verbindung (12), wobei das System (1) (i) eine Extraktionseinheit (100), (ii) eine Fließvorrichtung (400), die zur kontinuierlichen Entfernung von zumindest einem Teil einer ersten Flüssigkeit (10) aus einer ersten Entnahmezone (120) der Extraktionseinheit (100) ausgelegt ist, und (iii) einen Isomerisierungsreaktor (200), der einen mit der ersten Entnahmezone (120) der Extraktionseinheit (100) in Fluidkontakt stehenden Reaktoreinlass (210), einen mit der Mischzone (110) der Extraktionseinheit (100) in Fluidkontakt stehenden Reaktorauslass (220) und einen mit dem Reaktoreinlass (210) in Fluidkontakt und mit dem Reaktorauslass (220) in Fluidkontakt stehenden Fließkanal (230) umfasst, umfasst, wobei

- die Fließvorrichtung (400) zur Bereitstellung einer Zirkulationsströmung der ersten Flüssigkeit (10) von der ersten Entnahmezone (120) der Extraktionseinheit (100) über den Isomerisierungsreaktor (200) durch den Fließkanal (230) zu der Mischzone (110) der Extraktionseinheit (100) ausgelegt ist,
- der Isomerisierungsreaktor (200) einen Photoisomerisierungsreaktor umfasst,
- die Extraktionseinheit (100) eine Flüssig-Flüssig-Extraktionseinheit umfasst, die für das Inkontaktbringen der ersten Flüssigkeit (10) mit einer zweiten Flüssigkeit (20) ausgelegt ist, wobei die zweite Flüssigkeit (20) eine wässrige Lösung von Salpetersäure und Silbernitrat umfasst und wobei die erste Flüssigkeit (10) und die zweite Flüssigkeit (20) nicht miteinander mischbar sind und
- die Extraktionseinheit (100) Mischanlagen umfasst.

**12.** Verwendung des Systems (1) nach Anspruch 11, wobei das System (1) ferner (iv) eine Zuführvorrichtung für eine zweite Verbindung (99) umfasst, die mit der Mischzone (110) der Extraktionseinheit (100) in Fluidkontakt steht und zur Anreicherung der ersten Flüssigkeit (10) mit einer zweiten Verbindung (13) an einer der Extraktionseinheit (100) vorgeschalteten und dem Isomerisierungsreaktor (200) nachgeschalteten Stelle ausgelegt ist.

**13.** Verwendung des Systems (1) nach einem der Ansprüche 11-12, wobei die Extraktionseinheit (100) ferner einen Produktentnahmeanschluss (170) umfasst, der zur Entnahme von zumindest einem Teil der zweiten Flüssigkeit (20) aus der Extraktionseinheit (100) ausgelegt ist, wobei die Extraktionseinheit (100) ferner eine Zuführzone (140) umfasst, wobei das System (1) ferner eine zweite Flüssigkeitszuführvorrichtung (500), die in Fluidkontakt mit der Zuführzone (140) der Extraktionseinheit (100) angeordnet ist, umfasst.

**14.** Verwendung des Systems (1) nach einem der vorhergehenden Ansprüche 11-13, wobei das System (1) ferner ein Kühlsystem (300) umfasst, das zur Kühlung von zumindest einem Teil der ersten Flüssigkeit (10) in dem System (1) ausgelegt ist.

**15.** Verwendung des Systems (1) nach einem der vorhergehenden Ansprüche 11-14, wobei der Photoisomerisierungsreaktor zur Bereitstellung von UV-Licht, das aus dem Bereich von 240-270 nm ausgewählte Wellenlängen umfasst, ausgelegt ist, wobei zumindest ein Teil einer Wandung (231) des Fließkanals (230) für das UV-Licht durchlässig ist, wobei der Photoisomerisierungsreaktor ein UV-Leuchtelement (250) umfasst, das zur Bereitstellung des UV-Lichts ausgelegt ist, wobei zumindest ein Teil des Fließkanals (230) um das Leuchtelement (250) herum angeordnet ist.

## Revendications

**1.** Procédé pour une production continue d'un premier composé (12), le procédé comprenant l'alimentation en continu d'un premier liquide (10) à une zone de mélange (110) dans une unité d'extraction (100), le premier liquide (10) comprenant le premier composé (12) et un deuxième composé (13), le mélange dans la zone de mélange (110) du premier liquide (10) avec un deuxième liquide (20), le premier liquide (10) et le deuxième liquide (20) n'étant pas miscibles, le premier composé (12), en combinaison avec un troisième composé (22), possédant une solubilité plus élevée dans le deuxième liquide (20) que le deuxième composé (13) en combinaison avec le troisième composé (22), dans lequel dans une première zone de retrait (120) dans l'unité d'extraction (100), en contact fluidique avec la zone de mélange (110), au moins une partie du premier liquide (10) est éliminée en continu de l'unité d'extraction (100), le premier composé (12) étant un E-cyclooctène, le deuxième composé (13) étant un Z-cyclooctène, le premier liquide (10) étant un liquide organique (11), le deuxième liquide (20) étant un liquide aqueux (21), et le troisième composé (22) étant un sel métallique (23),

- le premier composé (12) étant un composé choisi dans le groupe constitué par les composés de formule (XI), (XII), (XIII), (XIV), (XV), et (XVI),

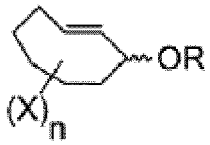
*(XI)*

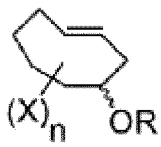
*(XII)*

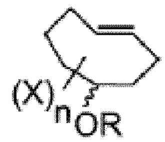
*(XIII)*

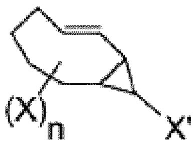
*(XIV)*

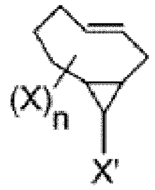
*(XV)*

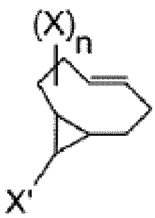
*(XVI)*

,

comprenant un cycle cyclooctène comprenant deux carbones joints à une double liaison :

◦ X et X' étant indépendamment choisis dans le groupe constitué par hydrogène, halogène, -OR, $-C(O)_2R^2$, $-NO_2$, -CN, $-S(O)_2R$, des groupes $C_{1-12}$-alkyle, des groupes $C_{1-12}$-aryle, des groupes $C_{1-12}$-alkylaryle, et des groupes $C_{1-12}$-arylalkyle, les groupes alkyle, les groupes aryle, les groupes alkylaryle et les groupes arylalkyle étant éventuellement substitués, et R étant indépendamment choisi dans le groupe constitué par hydrogène, halogène, des groupes $C_{1-24}$-alkyle, des groupes $C_{6-24}$-(hétéro) aryle, des groupes $C_{7-24}$-alkyl (hétéro) aryle et des groupes $C_{7-24}$-(hétéro)arylalkyle, les groupes alkyle, les groupes (hétéro)aryle, les groupes alkyl(hétéro)aryle et les groupes (hétéro)arylalkyle étant indépendamment éventuellement substitués par un ou plusieurs substituants indépendamment choisis dans le groupe constitué par des groupes $C_{1-12}$-alkyle, des groupes $C_{2-12}$-alcényle, des groupes $C_{2-12}$-alcynyle, des groupes $C_{3-12}$-cycloalkyle, des groupes $C_{1-12}$-alcoxy, des groupes $C_{2-12}$-alcényloxy, des groupes $C_{2-12}$-alcynyloxy, des groupes $C_{3-12}$-cycloalkyloxy, des halogènes, des groupes amino, des groupes oxo, et des groupes silyle, les groupes silyle étant représentés par la formule $(R^3)_3Si$-, les groupes alkyle, les groupes alcényle, les groupes alcynyle, les groupes cycloalkyle, les groupes alcoxy, les groupes alcényloxy, les groupes alcynyloxy et les groupes cycloalkyloxy étant éventuellement substitués, les groupes alkyle, les groupes alcoxy, les groupes cycloalkyle et les groupes cycloalcoxy étant éventuellement interrompus par un ou plusieurs hétéroatomes choisis dans le groupe constitué par O, N et S, et $R^3$ étant indépendamment choisi dans le groupe constitué par des groupes $C_{1-12}$-alkyle, des groupes $C_{2-12}$-alcényle, des groupes $C_{2-12}$-alcynyle, des groupes $C_{3-12}$-cycloalkyle, des groupes $C_{1-12}$-alcoxy, des groupes $C_{2-12}$-alcényloxy, des groupes $C_{2-12}$-alcynyloxy et des groupes $C_{3-12}$-cycloalkyloxy, les groupes alkyle, les groupes alcényle, les groupes alcynyle, les groupes cycloalkyle, les groupes alcoxy, les groupes alcényloxy, les groupes alcynyloxy et les groupes cycloalkyloxy étant éventuellement substitués, les groupes alkyle, les groupes alcoxy, les groupes cycloalkyle et les groupes cycloalcoxy étant éventuellement interrompus par un ou plusieurs hétéroatomes choisis dans le groupe constitué par O, N et S, et $R^2$ étant indépendamment choisi dans le groupe constitué par des groupes $C_{1-24}$-alkyle, des groupes $C_{6-24}$-(hétéro) aryle, des groupes $C_{7-24}$-alkyl(hétéro)aryle et des groupes $C_{7-24}$-(hétéro)arylalkyle, les groupes alkyle, les groupes (hétéro)aryle, les groupes alkyl(hétéro)aryle et les groupes (hétéro)arylalkyle étant éventuellement substitués ; X pouvant être situé au niveau d'une quelconque position sur le cycle cyclooctène autre qu'au niveau des deux carbones joints à la double liaison, et X étant éventuellement lié à un atome de carbone dans le groupe -OR, formant un groupe cyclique ; et
o (i) n étant au maximum 13, le premier composé (12) étant un composé choisi dans le groupe constitué par les composés de formule (XI), (XII), (XIII), ou (ii) n étant au maximum 12, le premier composé (12) étant un composé choisi dans le groupe constitué par les composés de formule (XIV), (XV), et (XVI), et

- le deuxième composé (13) comprenant un isomère du premier composé (12),
- le troisième composé (22) étant le nitrate d'argent,
- le deuxième liquide (20) comprenant le troisième composé (22) et une solution aqueuse d'acide nitrique, et

le procédé comprenant en outre la réintroduction du premier liquide (10) dans la zone de mélange (110) après soumission du premier liquide (10) à une étape d'isomérisation, le premier liquide (10) étant enrichi en premier composé (12), l'isomérisation dans l'étape d'isomérisation comprenant une photoisomérisation.

**2.** Procédé selon la revendication 1, le premier composé (12) étant un composé choisi dans le groupe constitué par un ester de cyclooctène et un cyclooctènediol protégé.

**3.** Procédé selon la revendication 2, l'ester de cyclooctène comprenant un alcanoate de cyclooctényle et/ou un cyclooctènecarboxylate d'organyle.

**4.** Procédé selon l'une quelconque des revendications précédentes, dans lequel dans une deuxième zone de retrait (130) dans l'unité d'extraction (100), en contact fluidique avec la zone de mélange (110), au moins une partie du deuxième liquide (20) est éliminée de l'unité d'extraction (100), le procédé comprenant en outre : l'alimentation du deuxième liquide (20) à l'unité d'extraction (100) dans une zone d'introduction (140) de l'unité d'extraction (100), la zone d'introduction (140) étant en contact fluidique avec la zone de mélange (110).

**5.** Procédé selon l'une quelconque des revendications précédentes, l'étape d'isomérisation comprenant la soumission du premier liquide à de la lumière UV, externe par rapport à l'unité d'extraction (100).

**6.** Procédé selon l'une quelconque des revendications précédentes, le premier liquide (10) comprenant en outre un sensibilisateur (15) choisi dans le groupe constitué par l'acétophénone, la benzophénone, le benzile, l'acridine, la duroquinone, le pyrène, l'éosine, et le benzoate de méthyle.

**7.** Procédé selon l'une quelconque des revendications précédentes, le premier liquide (10) étant enrichi en deuxième composé (13) à un endroit en aval d'un réacteur d'isomérisation (200) dans lequel ladite étape d'isomérisation a lieu, et en amont de ladite unité d'extraction (100).

**8.** Procédé selon l'une quelconque des revendications précédentes, le premier composé (12) comprenant un ou plusieurs cyclooctènes choisis dans le groupe constitué par le 10,10-diméthyl-9,11-dioxabicyclo [6.3.0]undécène, le bicyclo[6.1.0]nonène-9-carboxylate d'éthyle, l'acétoxybicyclo[6.1.0]nonène-9-carboxylate d'éthyle, l'acétate de cyclooctényle, et le diacétate de cyclooctényle.

**9.** Procédé selon l'une quelconque des revendications précédentes, le premier composé (12) comprenant un ou plusieurs cyclooctènes choisis dans le groupe constitué par les composés de formule (*VII*), (*VIII*), (*IX*), et (X) :

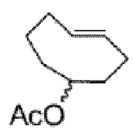

*(VII)*

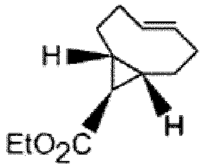

*(VIII)*

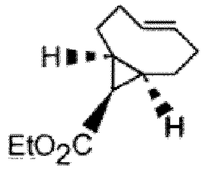

*(IX)*

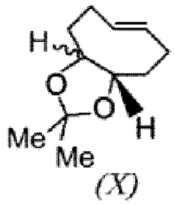

*(X)*

**10.** Procédé selon l'une quelconque des revendications précédentes, un pH du liquide aqueux étant choisi dans la plage de 3 à 5.

**11.** Utilisation d'un système (1) pour la mise en œuvre du procédé selon la revendication 1 pour une production continue d'un premier composé (12) tel que défini dans la revendication 1, le système (1) comprenant (i) une unité d'extraction (100), (ii) un dispositif d'écoulement (400) conçu pour éliminer en continu au moins une partie d'un premier liquide (10) d'une première zone de retrait (120) de l'unité d'extraction (100), et (iii) un réacteur d'isomérisation (200) comprenant une entrée de réacteur (210) en contact fluidique avec la première zone de retrait (120) de l'unité d'extraction (100), une sortie de réacteur (220) en contact fluidique avec une zone de mélange (110) de l'unité d'extraction (100) et un canal d'écoulement (230) en contact fluidique avec l'entrée du réacteur (210) et en contact fluidique avec la sortie du réacteur (220),

- le dispositif d'écoulement (400) étant conçu pour fournir un flux circulant du premier liquide (10) de la première zone de retrait (120) de l'unité d'extraction (100) via le réacteur d'isomérisation (200) à travers le canal d'écoulement (230) à la zone de mélange (110) de l'unité d'extraction (100),
- le réacteur d'isomérisation (200) comprenant un réacteur de photoisomérisation,
- l'unité d'extraction (100) comprenant une unité d'extraction liquide-liquide, conçue pour la mise en contact du premier liquide (10) avec un deuxième liquide (20), le deuxième liquide (20) comprenant une solution aqueuse d'acide nitrique et du nitrate d'argent et le premier liquide (10) et le deuxième liquide (20) étant immiscibles, et
- l'unité d'extraction (100) comprenant un moyen de mélange.

**12.** Utilisation du système (1) selon la revendication 11, le système (1) comprenant en outre (iv) un deuxième dispositif d'apport de composé (99) en contact fluidique avec la zone de mélange (110) de l'unité d'extraction (100) et conçu pour enrichir le premier liquide (10) en un deuxième composé (13) à un endroit en amont de l'unité d'extraction (100) et en aval du réacteur d'isomérisation (200).

**13.** Utilisation du système (1) selon l'une quelconque des revendications 11 et 12, l'unité d'extraction (100) comprenant en outre un orifice de retrait de produit (170) conçu pour le retrait d'au moins une partie du deuxième liquide (20) de l'unité d'extraction (100), l'unité d'extraction (100) comprenant en outre une zone d'introduction (140), le système (1) comprenant en outre un deuxième dispositif d'apport de liquide (500) agencée en contact fluidique avec ladite zone d'introduction (140) de l'unité d'extraction (100).

**14.** Utilisation du système (1) selon l'une quelconque des revendications précédentes 11 à 13, le système (1) comprenant en outre un système de refroidissement (300) conçu pour refroidir au moins une partie du premier liquide (10) dans le système (1).

**15.** Utilisation du système (1) selon l'une quelconque des revendications précédentes 11 à 14, le réacteur de photoisomérisation étant conçu pour fournir de la lumière UV comprenant une longueur d'onde choisie dans la plage de 240 à 270 nm, au moins une partie d'une paroi (231) du canal d'écoulement (230) étant transmissive pour ladite lumière UV, le réacteur de photoisomérisation comprenant un élément d'éclairage UV (250) conçu pour fournir ladite lumière UV, au moins une partie du canal d'écoulement (230) étant agencée de sorte à entourer l'élément d'éclairage (250).

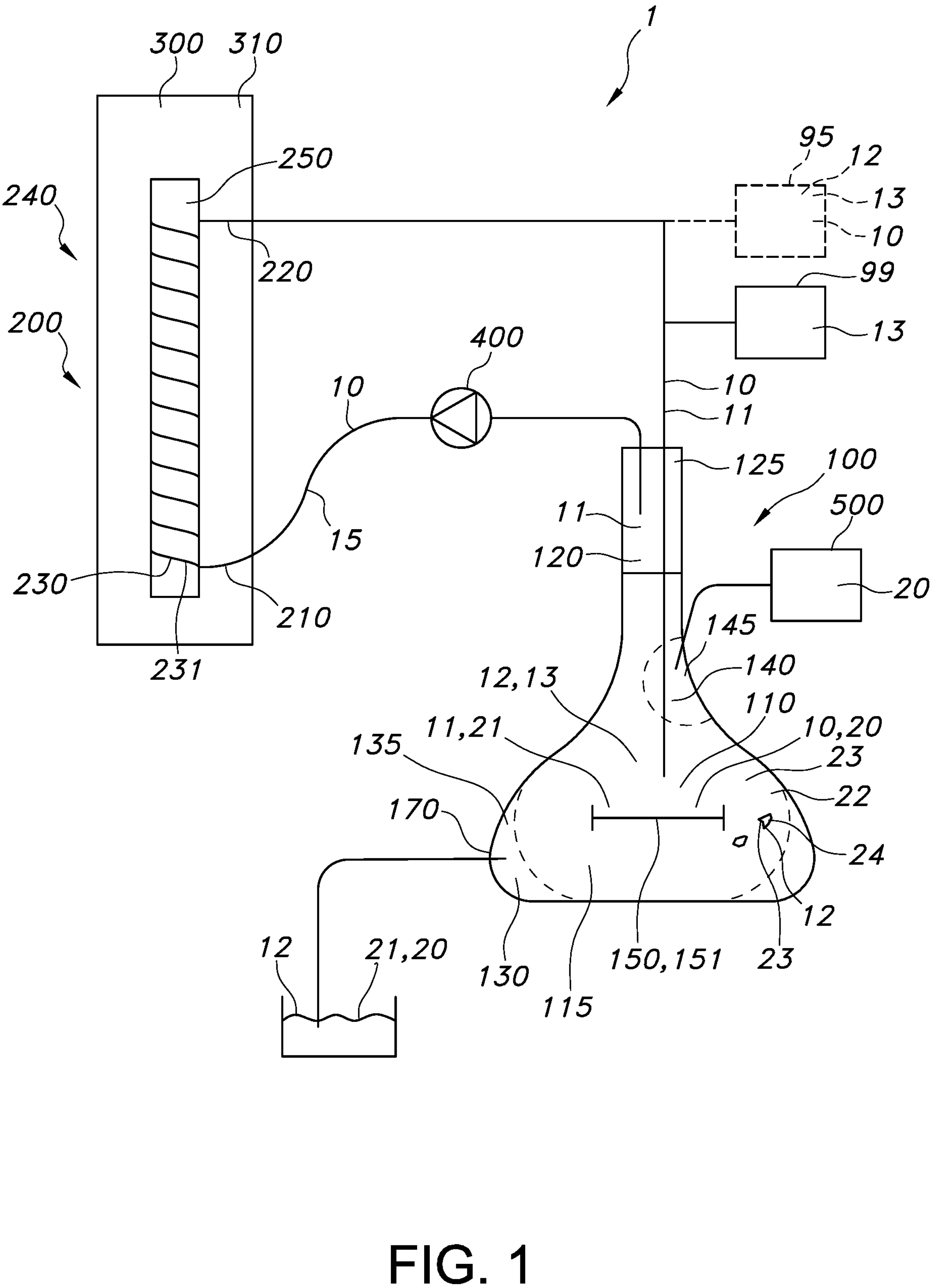
1
300
310
250
240
220
95
12
13
10
99
13
200
400
10
10
11
125
100
11
15
500
120
230
210
20
231
145
140
12,13
110
11,21
10,20
135
23
22
170
24
12
150,151
23
130
12
21,20
115

FIG. 1

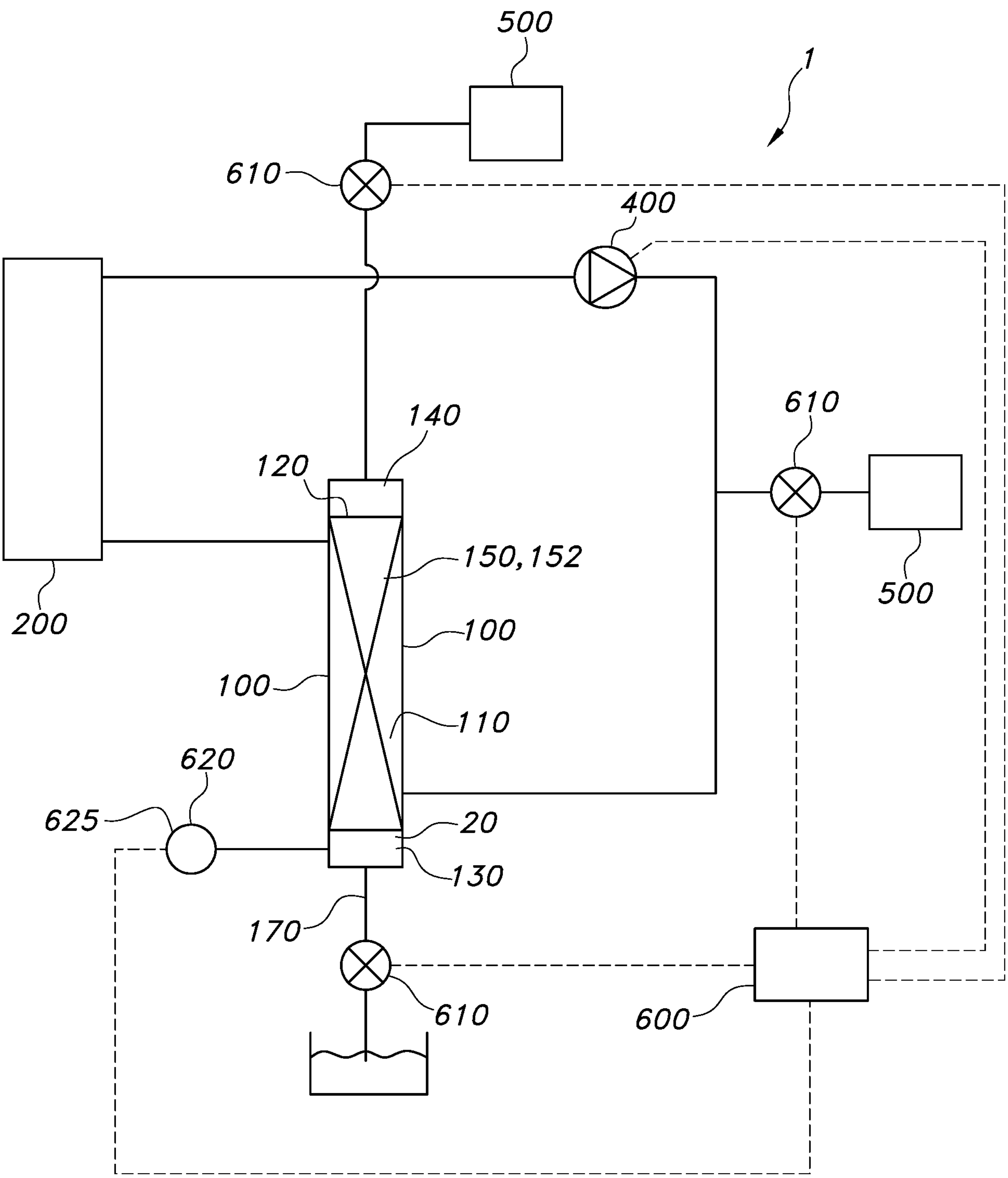
500
1
610
400
140
610
120
150,152
500
200
100
100
110
620
625
20
130
170
610
600

FIG. 2

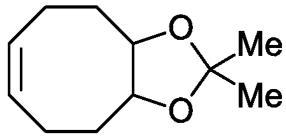
(I)

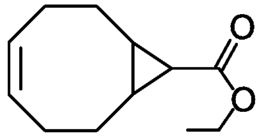
(II)

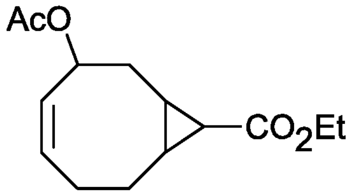
(III)

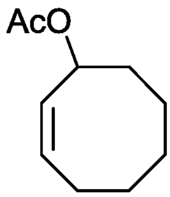
(IV)

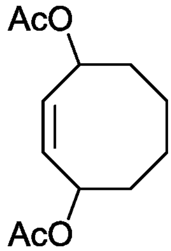
(V)

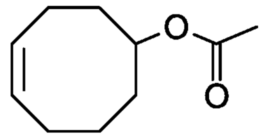
(VI)

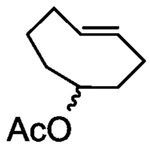
(VII)

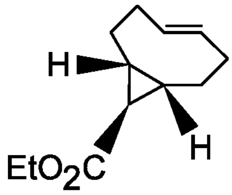
(VIII)

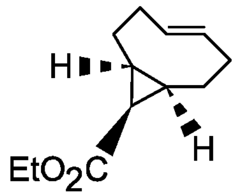
(IX)

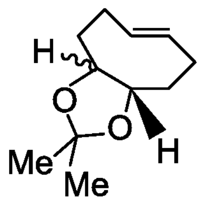
(X)

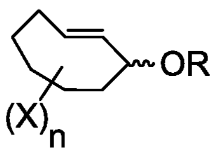
(XI)

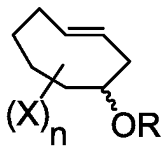
(XII)

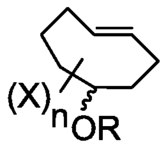
(XIII)

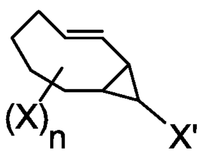
(XIV)

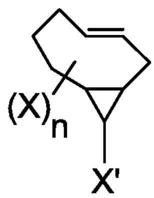
(XV)

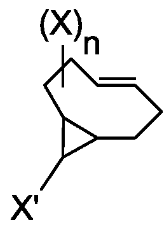
(XVI)

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2894142 A1


### Non-patent literature cited in the description


- **M. ROYZEN et al.** *Journal of the American Chemical Society,* 2008, vol. 130, 3760-3761
- **ARTHUR C. COPE et al.** trans-cyclooctene. *Organic synthesis,* 1969, vol. 49, 39-40